(19) **Europäisches Patentamt / European Patent Office / Office européen des brevets**

(11) **EP 3 583 249 B1**

(12) **EUROPEAN PATENT SPECIFICATION**

(45) Date of publication and mention
of the grant of the patent:
**19.07.2023 Bulletin 2023/29**

(21) Application number: **17896453.2**

(22) Date of filing: **14.12.2017**

(51) International Patent Classification (IPC):
**G16B 50/50** *(2019.01)* **G16B 30/20** *(2019.01)*
**H03M 7/30** *(2006.01)* **G06F 21/62** *(2013.01)*

(52) Cooperative Patent Classification (CPC):
**H03M 7/3086; G06F 21/6245; G16B 30/20; G16B 50/50; H03M 7/30; H03M 7/70**

(86) International application number:
**PCT/US2017/066458**

(87) International publication number:
**WO 2018/151786 (23.08.2018 Gazette 2018/34)**

(54) **METHOD AND SYSTEMS FOR THE RECONSTRUCTION OF GENOMIC REFERENCE SEQUENCES FROM COMPRESSED GENOMIC SEQUENCE READS**

VERFAHREN UND SYSTEME ZUR REKONSTRUKTION VON GENOMREFERENZSEQUENZEN AUS KOMPRIMIERTEN GENOMSEQUENZABSCHNITTEN

PROCÉDÉ ET SYSTÈMES DE RECONSTRUCTION DE SÉQUENCES DE RÉFÉRENCE GÉNOMIQUES À PARTIR DE LECTURES DE SÉQUENCES GÉNOMIQUES COMPRESSÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.02.2017 PCT/US2017/017842**
**11.07.2017 PCT/US2017/041579**

(43) Date of publication of application:
**25.12.2019 Bulletin 2019/52**

(73) Proprietor: **Genomsys SA**
**1015 Lausanne (CH)**

(72) Inventors:
• **BALUCH, Mohamed Khoso**
**Chantilly, Virginia 20151 (US)**
• **ALBERTI, Claudio**
**1213 Petit-Lancy (CH)**

(74) Representative: **Metroconsult Srl**
**Foro Bonaparte 51**
**20121 Milano (IT)**

(56) References cited:
• **Mihai Pop ET AL: "Comparative genome assembly", Briefings in bioinformatics, 1 September 2004 (2004-09-01), pages 237-248, XP55742204, England DOI: 10.1093/bib/5.3.237 Retrieved from the Internet: URL:http://ccb.jhu.edu/people/salzberg/doc s/AMOScmp-reprint.pdf**
• **K. SCHNEEBERGER ET AL: "Reference-guided assembly of four diverse Arabidopsis thaliana genomes", PROCEEDINGS OF THE NATIONAL ACADEMY OF SCIENCES, vol. 108, no. 25, 21 June 2011 (2011-06-21), pages 10249-10254, XP055222736, ISSN: 0027-8424, DOI: 10.1073/pnas.1107739108**
• **Anonymous: "CRAM format specification (version 3.0)", , 8 September 2016 (2016-09-08), XP002771305, Retrieved from the Internet: URL:https://samtools.github.io/hts-specs/C RAMv3.pdf [retrieved on 2017-06-22]**
• **TATWAWADI et al.: "GTRAC: fast retrieval from compressed collections of genomic variants", Bioinformatics, vol. 32, no. 17 1 September 2016 (2016-09-01), pages i479-i486, XP055535444, Retrieved from the Internet: URL:doi: 10.1093/bioinformatics/btw437**

**(Cont. next page)**

EP 3 583 249 B1

- DAILY et al.: "Data structures and compression algorithms for high-throughput sequencing technologies", BMC Bioinformatics, vol. 11, 14 October 2010 (2010-10-14), pages 1-12, XP021071841,
- BAO et al.: "AlignGraph: algorithm for secondary de novo genome assembly guided by closely related references. AlignGraph: algorithm for secondary de novo genome assembly guided by closely related references", Bioinformatics, vol. 30, no. 12 15 June 2014 (2014-06-15), pages i319-i328, XP055535457, Retrieved from the Internet: URL:https://doi.org/10.1093/bioinformatics /btu291
- PANNEEL: "Implementation and optimization of CABAC in a genomic data compression framework with support for random access", Department of Electronics and Information Systems, August 2015 (2015-08), XP055535469, Retrieved from the Internet: URL:https://lib.ugent.be/tulltxt/ROG01/002 /224/797/RUG01-002224797 _2015_000 1_AC.pdf [retrieved on 2018-03-27]

**Description**

**TECHNICAL FIELD**

This disclosure relates to the lossless compression of aligned genomic sequence reads with the associated alignment information and the reference genomes, or portions thereof, used to align said genomic sequences. A genomic sequence is generally intended as a concatenation of molecules called nucleotides to form fragments of deoxyribonucleic acid (DNA) or Ribonucleic acid (RNA). The present invention can be applied to any reference sequence of symbols used to align shorter sequences using the same alphabet.

The present invention applies to aligned genomic sequences which have been compressed by means of a reference-less compression method. An initial attempt in this direction is the one described in Voges, J., Munderloh, M., Ostermann, J., "Predictive Coding of Aligned Next-Generation Sequencing Data" (2016 Data Compression Conference (DCC)) or the in Benoit, G., et al. "Reference-free compression of high throughput sequencing data with a probabilistic de Bruijn graph" (BMC Bioinformatics. 2015; 16: 288.) with, however, several limitations addressed in the present invention.

In the context of this disclosure, reference-less compression of aligned genomic sequences comprises the creation of one or more local reference sequences named "contig" built by overlapping and concatenating genomic sequences mapped in adjacent or overlapping regions of the reference genome used for alignment. For an exhaustive description of contig see https://en.wikipedia.org/wiki/Contig. Said contigs do not need to be included in the compressed bitstream since they are reconstructed at the decoding end as part of the decoding process. Once a contig has been built for a genomic region where one or more genomic sequences have been mapped, reference-based compression is applied to said genomic sequences by describing them in terms of genomic descriptors and compressing each block of genomic descriptors of the same type with a specific entropy coder. This approach enables reaching better compression ratios than general purpose compression schemes such as GZIP, LZMA, BZ and preserves random access.

Reference-based compression of aligned genomic sequences is based on representing said aligned sequences in terms of their mapping positions and differences with respect to the one or more reference sequences used for alignment and encoding only said positions and differences. Whereas such approach permits to reach very high compression ratios (roughly linearly increasing with the coverage, wherein with the term coverage is intended the average number of reads which contain each nucleotide of a reference genome), both the encoding and decoding process require the availability of the specific reference sequences used for the alignment and the compression. A drawback of the approach is that if the reference sequence used for the alignment and the compression is not available at the decoding side (for example due to the lack of a unique identification of the reference genome or its version, or in case the original data source is no more available), the compressed content cannot be recovered. Solutions based on including the reference genomes in the compressed representation for storage or transmission would result to be detrimental in terms of compression efficiency.

To address such problem, reference-less compression methods enabling compression and decompression of aligned genomic sequence reads without using the reference genome used for alignment do exist. Some of these methods adopt general purpose compressors such as GZIP, BZIP2, LZMA and reach poor compression ratios in the order to 3:1. More efficient methods are based on the construction of one or more reference sequences from the aligned reads themselves by means of a process called "assembly" in which reads mapped on adjacent genomic intervals of the reference genome used for alignment are used to build longer sequences by finding shared subsequences and concatenating them. The longer sequence obtained from the concatenation or merge of shorter ones is called "contig". Such methods include the already cited publication from Voges, J., Munderloh, M., Ostermann, J., "Predictive Coding of Aligned Next-Generation Sequencing Data" (2016 Data Compression Conference (DCC)) and the paper from Benoit, G., et al. "Reference-free compression of high throughput sequencing data with a probabilistic de Bruijn graph" (BMC Bioinformatics. 2015; 16: 288.).

This disclosure addresses the problem of the efficient compression of the reference genome used for the alignment of genomic sequence reads when jointly applying reference-less compression of genomic sequences.

Document Mihai Pop ET AL: "Comparative genome assembly", Briefings in bioinformatics, 1 September 2004, pages 237-248, discloses the AMOS Comparative Assembler (AMOS-Cmp), where the overlap step is skipped entirely, and instead, the reads are aligned to the reference genome using a modified version of the MUMmer algorithm.

Document K. SCHNEEBERGER ET AL: "Reference-guided assembly of four diverse Arabidopsis thaliana genomes", PROCEEDINGS OF THE NATIONAL ACADEMY OF SCIENCES, vol. 108, no. 25, 21 June 2011, pages 10249-10254, discloses reads and their alignments; specifically, adjacent blocks are combined into superblocks being defined in an overlapping manner, such that blocks can belong to several superblocks. All reads of a single superblock are assembled with reads that are not aligned. Contigs resulting therefrom are merged into a non-redundant set of supercontigs.

Document "CRAM format specification (version 3.0)", 8 September 2016, discloses entropy encoding and storing information about the sequence under use of specific formats, in particular CRAM format. CRAM is a reference-based compression toolkit, employing GZIP.

## SUMMARY

In a first aspect, the invention refers to a computer-implemented method for encoding aligned genome sequence data comprising reads of sequences of nucleotides according to claim 1.

Preferably, assembling said aligned reads comprises the step of selecting, for each position on the reference sequence, the nucleotide that is present with the highest frequency in the aligned reads at that position.

Preferably, the length of said contig is defined as input parameter to the encoder or dynamically adapted by the encoder.

Preferably, said first descriptor is binarized using a Split Unit-wise Truncated Unary binarization, wherein said Split Unit-wise Truncated Unary is a concatenation of repeated truncated unary binarizations, where each truncated unary binarization is applied to portions of the value to be binarized which are N bits long, wherein N is a preselected parameter.

Preferably, said second descriptor is binarized using a Truncated Unary binarization, wherein the value of said second descriptor is followed by a zero and if said value is equal to the largest possible value to be binarized the trailing 0-bit is discarded.

Preferably, said method does not encode information signalling the usage of a specific reference sequence. Preferably, said length of said contig is contained in a syntax header.

In a second aspect, the invention refers to an apparatus for encoding genome sequence data, said genome sequence data comprising reads of sequences of nucleotides, according to claim 6.

Preferably, said means for assembling said aligned reads further comprise means for selecting, for each position on the reference sequence, the nucleotide that is present with the highest frequency in the aligned reads at that position.

Preferably, the apparatus for encoding genome sequence data, further comprises means to receive the length of said contig as input parameter and means for dynamically adapting length of said contig.

Preferably, the apparatus for encoding genome sequence data, further comprises binarization means for binarizing said first descriptor by employing a Split Unit-wise Truncated Unary binarization, wherein said Split Unit-wise Truncated Unary is a concatenation of repeated truncated unary binarizations, where each truncated unary binarization is applied to portions of the value to be binarized which are N bits long, wherein N is a preselected parameter.

Preferably, the apparatus for encoding genome sequence data, further comprises binarization means for binarizing said second descriptor by employing a Truncated Unary binarization, wherein the value of said second descriptor is followed by a zero and if said value is equal to the largest possible value to be binarized the trailing 0-bit is discarded.

Preferably, the apparatus for encoding genome sequence data, further comprises means for coding said length of said contig in a syntax header.

In a third aspect, the invention refers to a computer-implemented method for decoding encoded genome sequence data, according to claim 8.

Preferably, the decoding method further comprises decoding the length of said contig from a syntax header contained in the input file.

Preferably, Preferably the decoding method runner comprises a reverse omarization of said first descriptor, wherein said first descriptor is binarized using a Split Unit-wise Truncated Unary binarization, wherein said Split Unit-wise Truncated Unary is a concatenation of repeated truncated unary binarizations, where each truncated unary binarization is applied to portions of the value to be binarized which are N bits long, wherein N is a preselected parameter

Preferably, the decoding method further comprises a reverse binarization of said second descriptor wherein said second descriptor is binarized using a Truncated Unary binarization, wherein the value of said second descriptor is followed by a zero and if said value is equal to the largest possible value to be binarized the trailing 0-bit is discarded.

Preferably, said input file does not contain information signalling the usage of a specific reference sequence.

In a fourth aspect, the invention refers to an apparatus for decoding encoded genome sequence data according to claim 12.

Preferably, the apparatus for decoding encoded genome sequence data, further comprises means for decoding from a syntax header contained in the input file the information relating to the length of said contig.comprises

Preferably, the apparatus for decoding encoded genome sequence data, further comprises means for a reverse binarization of said first descriptor, wherein said first descriptor is binarized using a Split Unit-wise Truncated Unary binarization, wherein said Split Unit-wise Truncated Unary is a concatenation of repeated truncated unary binarizations, where each truncated unary binarization is applied to portions of the value to be binarized which are N bits long, wherein N is a preselected parameter.

Preferably, the apparatus for decoding encoded genome sequence data, further comprises means for a reverse binarization of said second descriptor wherein said second descriptor is binarized using a Truncated Unary binarization, wherein the value of said second descriptor is followed by a zero and if said value is equal to the largest possible value to be binarized the trailing 0-bit is discarded.

**BRIEF DESCRIPTION OF THE DRAWINGS**

Figure 1 shows how genomic sequences are mapped on a reference sequence and then used to build an assembly by means of merging and concatenation operations. The constructed assembly can differ from the reference sequence at positions where the aligned genomic sequences present different nucleotides than those present in the reference sequence.

Figure 2 shows how he mismatches between the reference sequence and the contig are represented in terms of positions and type of mismatch and then encoded using entropy coders implementing different binarizations and transformations as defined in this disclosure.

Figure 3 depicts an encoder apparatus comprising the steps of aligning genomic sequences with respect to a reference genome, constructing contigs by merging and concatenating said aligned sequences, generating descriptors representing the genomic sequences with respect to the contigs, compressing each block of descriptors with a dedicated entropy coder.

Figure 4 shows the decoding process of a compressed bitstream comprising the steps of demultiplexing the incoming bitstream to extract the entropy coded descriptors, entropy decoding of each type of descriptors, construction of contigs, decoding of aligned sequence reads using the constructed contigs, reconstruction of the reference genome using the contigs and the contig mismatch positions and types.

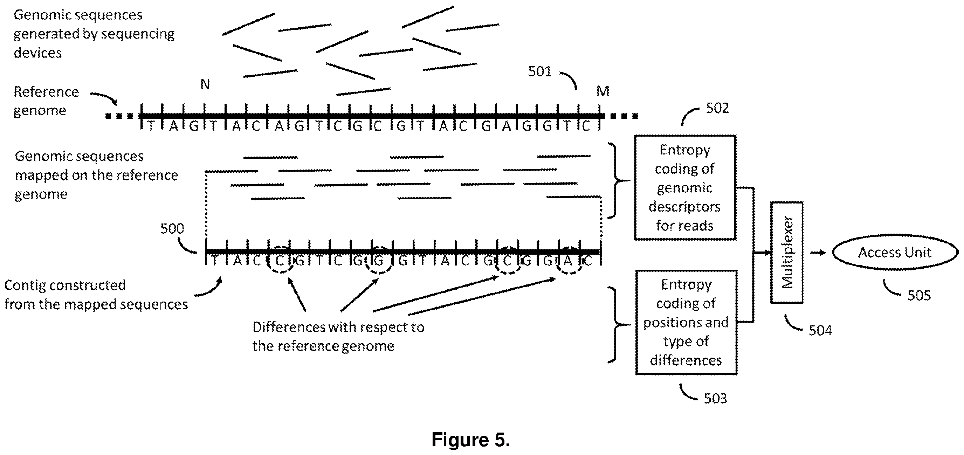

Figure 5 shows how sequence reads mapped between coordinate N and coordinate M on the reference sequence are used to build a contig of length M-N nucleotides. Reference-based compression is then applied to the mapped sequence reads using the constructed contig. Genomic descriptors representing the mapped sequence reads are entropy coded and multiplexed in the same Access Unit as the entropy coded genomic descriptors representing the differences between the reference sequence used for alignment and the constructed contig.

Figure 6 shows how an Access Unit encapsulates compressed descriptors representing sequence reads mapped in a contiguous interval of the reference sequence. Header information is prepended to the compressed descriptors in order to enable data parsing.

Figure 7 shows how an Access Unit of type P is composed by a header and the multiplexing of blocks of descriptors representing the reads mapping positions (pos), the reverse complement information (rcomp), the pairing information in case of paired end reads (pair), the reads length in case of variable reads length (rlen) and mapping flags (flags). It is used to encode reads of class P

Figure 8 shows a coordinate system on a Reference Sequence and mapping of reads and read pairs on a Reference Sequence.

Figure 9 shows how the unmapped mates in read pairs can be assembled to build a contig that can fill the gap in a reference sequence. Additionally, previously unmapped read pairs can then be mapped to the newly assembled contig.

Figure 10 shows an example of transformation and binarization of five mismatches between a reference genome and an assembled contig.

Figure 11 shows how regions of the reference genome used for alignment where no sequence reads are mapped are coded in dedicated Access Units in order to enable complete reconstruction of the reference genome at the decoding end.

**DETAILED DESCRIPTION**

The genomic or proteomic sequences referred to in this invention include, for example, and not as a limitation, nucleotide sequences, Deoxyribonucleic acid (DNA) sequences, Ribonucleic acid (RNA), and amino acid sequences. Although the description herein is in considerable detail with respect to genomic information in the form of a nucleotide sequence, it will be understood that the methods and systems for compression can be implemented for other genomic or proteomic sequences as well, albeit with a few variations, as will be understood by a person skilled in the art.

Genome sequencing information is generated by High Throughput Sequencing (HTS) machines in the form of sequences of nucleotides (a. k. a. "bases") represented by strings of letters from a defined vocabulary. The smallest vocabulary is represented by five symbols: {A, C, G, T, N} representing the 4 types of nucleotides present in DNA namely Adenine, Cytosine, Guanine, and Thymine. In RNA Thymine is replaced by Uracil (U). N indicates that the sequencing machine was not able to call any base and so the real nature of the nucleotide at that position is undetermined. In case the IUPAC ambiguity codes are adopted by the sequencing machine as vocabulary, the alphabet used for the symbols is composed of the following symbols: {A, C, G, T, U, W, S, M, K, R, Y, B, D, H, V, N or -}. In case of amino acids the supported symbols are: {A, C, D, E, F, G, H, I, K, L, M, N, P, Q, R, S, T, V, W, Y}.

**Terminology**

In the context of this disclosure the nucleotides sequences produced by sequencing machines are called **reads.** Sequence reads can be composed of a number of nucleotides ranging between a few dozens to several thousand. Some sequencing technologies produce sequence reads composed of **pairs** of which one read is originated from one DNA strand and the other is originated from the other strand. A read associated to another read in a sequencing process producing pairs is said to be its **mate.**

A common element of efficient approaches to genomic sequence reads compression is the exploitation of the correlation of sequence data with respect to a reference sequence. Even if the somatic profile of the human population is extremely diversified, the actual portion of the number of nucleotides that differs from person to person is about only 0.1% of the total number of nucleotides composing an entire genome. Therefore, the specific genomic information characterizing each individual is very limited with respect to the entire information carried by an entire genome. When a pre-existing reference genome is available, be it for previous sequencing or as a published "average" consensus reference, the most common way as of today to encode the information is to identify and encode only the differences with respect to the reference genome.

In order to do so with raw sequence reads, generally expressed in the form of FASTQ data files, a preliminary pre-processing step the mapping on a reference genome. In case an appropriate reference genome is not available, or if the bias introduced by the usage of a specific reference is not desirable, the construction of a new reference sequence by means of assembling the sequence reads at hand into longer sequences called **contigs,** is a possible alternative.

Throughout this disclosure, a **reference sequence** is a sequence of nucleotides associated to a mono-dimensional integer coordinate system for which each integer coordinate is associated to a single nucleotide. Coordinate values can only be equal or larger than zero. This coordinate system in the context of this invention is zero-based (i.e. the first nucleotide has coordinate 0 and it is said to be at position 0) and linearly increasing from left to right.

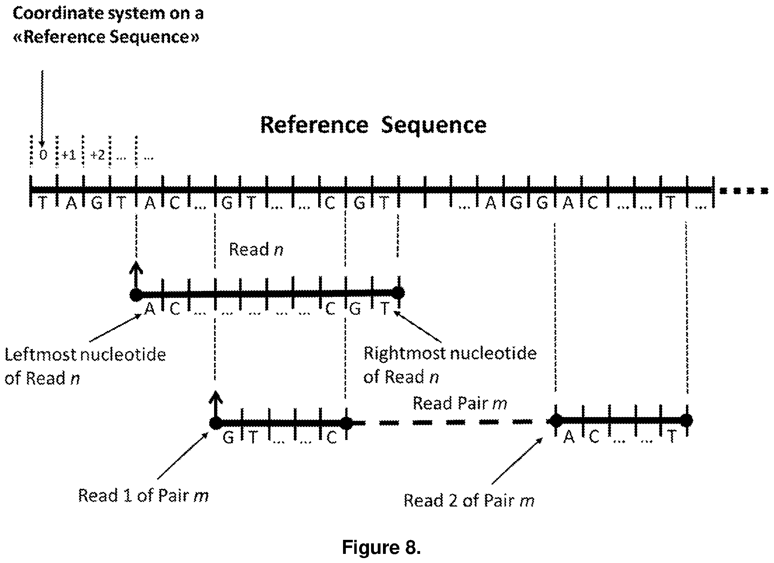

When mapping sequence reads on a reference sequence, said reference sequence is used as axis of a mono-dimensional coordinate system in which the left-most position is denoted as position 0. For each sequence read, mapped to a reference sequence, the nucleotide mapped at the reference sequence position identified by the smallest coordinate number is usually referred to as the "left-most" nucleotide, whereas the nucleotide mapped at the reference sequence position identified by the largest coordinate number is referred to as the "right-most" nucleotide. This is illustrated in Figure 8. Throughout this disclosure, a nucleotide is also referred to as a **base.**

When a sequence read is mapped to a reference sequence, the coordinate of the left-most mapped base is said to represent the **mapping position** of the read on the reference sequence.

A base present in the aligned read and not present in the reference sequence (a.k.a. insertion) and bases preserved by the alignment process but not mapped on the reference sequence (a.k.a. soft clips) do not have mapping positions.

When a sequence read cannot be mapped to any mapped position of the used reference sequences according to the specified matching rules, it is said to be **unmapped.**

The process of building longer genomic sequences by looking for overlapping regions among sequence reads is called **assembly.**

A longer genomic sequence built assembling shorter reads is called **contig** (see https://en.wikipedia.org/wiki/Contig).

Sequence reads that fail to build any contig during an assembly process are said to be **unaligned.**

A **reference genome** is composed by one or more reference sequences and it is assembled by scientists as a representative example of a species' set of genes. For example GRCh37, the Genome Reference Consortium human genome (build 37) is derived from thirteen anonymous volunteers from Buffalo, New York. However, a reference sequence could also consist of a synthetic sequence conceived and merely constructed to improve the compressibility of the reads in view of their further processing.

In this disclosure the read composing a read pair with a base mapping on the smallest coordinate on a reference sequence is referred to as "**Read 1**" whereas its mate is referred to as "**Read 2**".

The distance, expressed as number of nucleotides (or bases), separating two reads generated as a pair, by a sequencing machine using current state of the art sequencing technology, is unknown, and it is determined by mapping both reads composing the pair (i.e. minimizing appropriate matching functions) to a reference sequence.

Throughout this disclosure, an **Access Unit** (AU) is defined as a logical data structure containing a coded representation of genomic information or related metadata to facilitate the bit stream access and manipulation. It is the smallest data organization that can be decoded by a decoding device implementing the invention described in this disclosure. According to the type of coded information, an AU can be decoded either independently of any other AU or using information contained in other AUs.

AUs can be classified into a multiplicity of types according to the nature of the coded sequence data. An Access Unit contains either a reference sequence, or a portion thereof, or encoded reads or read pairs belonging to a single class of data. Any single AU cannot contain two or more types of sequence data. For example an Access Unit may contain the entire chromosome 1 of GRCh37, the Genome Reference Consortium human genome (build 37). Another Access Unit may contain the coded representation of nucleotides of chromosome 1 of GRCh37 that are located between coordinates 50'000 and 150'000. Another Access Unit may contain only reads or read pairs that perfectly map on the reference sequence without any mismatch. Another Access Unit may contain reads or read pairs that only contain "N" symbols as mismatches with respect to the reference sequence. Another Access Unit may contain reads or read pairs that contain any type of substitutions (e.g. one base present in the read or read pair is different from the base at the corresponding mapping position in the reference sequence). Another Access Unit may contain reads or read pairs that contain mismatches, insertions, deletions and soft clipped bases. Another Access Unit may contain only read or read pairs that do not map on the reference sequence. Another Access Unit may contain only read pairs in which one read is mapped and the other is not mapped on the reference sequence. Another type of Access Unit may contain only encoded segments of a reference genome composed by one or more reference sequences (for example chromosomes).

The essential characteristic of an Access Unit is that it contains in compressed form all elements needed to reconstruct the genomic information of sequence reads or read pairs, reference sequences, associated alignment information and metadata of reads or read pairs. In other words, to fully reconstruct the reads, or the read pairs, or the reference sequence and the associated information carried by an Access Unit it is only necessary to retrieve and decompress the Access Unit itself and, when applicable, the Access Units containing the reference sequence to which the Access Units refers to.

In each Access Unit the descriptors listed in the next section and representing the information on encoded read or read pairs, are aggregated in separate data blocks - one per type - in order to exploit their homogeneous statistical properties for achieving high performance entropy coding.

Each Access Unit contains the compressed sub-set of descriptors representing sequence reads or read pairs belonging to the same data class mapped to a genomic region on a reference sequence. Such genomic region on the reference sequence is defined by a start coordinate (or start position) and an end coordinate (or end position).

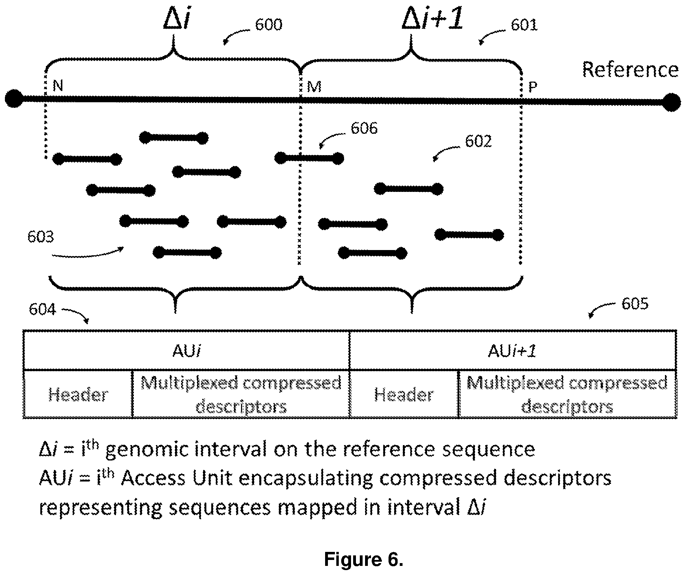

An example of Access Unit is illustrated in Figure 6. Access Units are composed by blocks of encoded genomic descriptors as described in the next section. To enable transport over a network, blocks are further decomposed into packets. When compressing genomic sequence reads, each Access Units contains compressed descriptor representing either sequence reads mapped to a genomic interval on the reference sequence or unmapped sequence reads. Access Units can be used to carry reference genomes or parts thereof. A reference sequence can be either encoded as a single long sequence of nucleotides or split into shorter sequences encoded as unmapped genomic sequence reads.

In the context of this disclosure, **genomic descriptors** are syntax elements representing part of the information (and also elements of a syntax structure of a file format and/or a bitstream) necessary to reconstruct (i.e. decode) coded reference sequences, sequence reads and the associated mapping information.

The genomic descriptors disclosed in this invention are listed in Table 1.

**Table 1. Genomic descriptors and their meaning**

| ID | short name | description |
|---|---|---|
| 1 | pos | the mapping position of a read on a reference sequence |
| 2 | pair | the distance between a read and its mate, |
| 3 | rlen | the length of a sequence read |
| 4 | rcomp | the DNA or RNA strand the reads was mapped on |

(continued)

| ID | short name | description |
|---|---|---|
| 5 | mmpos | the position of mismatches (i.e. substitutions, deletion and insertions ) in aligned reads with respect to reference sequences |
| 6 | mmtype | the types of mismatches with respect to reference sequences at the associated positions |
| 7 | clips | the bases that could not be mapped on the reference sequence by the mapping procedure and either kept ("soft clipped" bases) or discarded ("hard clipped" bases) |
| 8 | flags | the mapping flags enabling the aligner to further specify the result of the mapping process such as if the sequence read is a PCR or optical duplicate |
| 9 | mmap | the multiple mapping positions that are associated to a single read or read pair by the mapping procedure |
| 10 | msar | the identification of the existence of spliced reads (i.e. reads that when split in chunks find mapping positions with higher degrees of matching accuracy than when they are mapped as single connected read mapped on a single position on a reference sequence) |
| 11 | ureads | the representation of sequence reads that cannot be mapped at any position of the reference sequence with specified degrees of matching accuracy, |
| 12 | rtype | the descriptor signaling the subset of descriptors used to encode sequence reads that cannot be mapped at any position of the reference sequence with specified degrees of matching accuracy |
| 13 | rftp | the position of mismatches between a contig and a reference sequence. Positions of mismatches are terminated by a special terminator character. |
| 14 | rftt | the type of mismatches between a contig and a reference sequence. |
| 15 | rgroup | the label associated to each compressed sequence read used to create group of reads sharing the same label |
| 16 | mscore | the score per alignment. It is used to represent mapping/alignment score per read generated by genomic sequence reads aligners. |

According to the method disclosed in this invention, reference sequences or portion thereof, sequence reads and the associated alignment information are coded using a sub-set of the descriptors listed above which are then entropy coded using a multiplicity of entropy coders according to each descriptor specific statistical properties. Blocks of compressed descriptors with homogeneous statistical properties are structured in Access Units which represent the smallest coded representation of one or more genomic sequence that can be manipulated by a device implementing the invention described in this disclosure.

The invention described in this disclosure defines a method to represent in terms of genomic descriptors a reference sequence used to aligning genomic sequence reads. Such genomic descriptors are compressed using specific entropy coders. The blocks of compressed descriptors are then encapsulated in the same Access Unit containing the compressed genomic descriptors enabling reconstruction of sequence reads mapped on said reference sequence so as to enable efficient transport and selective access.

In an embodiment, the present principles are directed to the lossless compression of both genomic sequence reads with associated alignment information and reference sequences used for alignment. Efficient compression is obtained by building longer sequences called contigs by merging or concatenating sequence reads mapped in contiguous regions of the reference sequence and then performing reference-based compression of sequence reads with respect to the constructed contig. Better compression is obtained by using separate syntax elements called "genomic descriptors" to represent all the features characterizing genomic sequence reads mapped on a reference sequence. The SAM format stores alignment information with the nucleotides sequence and other metadata such as the quality scores associated to base pairs, pairing information in case of paired end reads, and so forth. According to the principles of the present invention, each of the features associated to a mapped or unmapped genomic sequence read is represented by a genomic descriptor. Genomic descriptors of the same type are concatenated in blocks which are compressed using different entropy coders. It has to be appreciated that the present invention does not compress any of the SAM fields such as the CIGAR string, the mapping flags, nucleotides strings or quality values strings, but represent the same type of information using the 16 genomic descriptors or specific sub-sets of them. Round-trip transcoding to/from SAM and the presented format is possible for the eleven mandatory fields of SAM.

State of the art solutions store assembled genomes in the form of compressed FASTA files where each reference sequence (e.g. chromosomes) is stored as a sequence of symbols representing the four nucleotides. Each reference sequence is part of a reference genome (a.k.a. genome assembly) which is compressed and stored in a database. Several genome assemblies obtained at different moments in time from organic material belonging to the same organism or individual are stored to represent its genetic history. When genomic analysis requires comparing genome sequence data with existing reference genomes, the genomic analysis applications perform queries to a database to retrieve the one or more reference genomes of interest and perform the genomic analysis.

In a preferred embodiment, the principles disclosed in this invention can be applied to implement efficient storage of collections of reference genomes embedded in compressed genome sequence data without the need of storing the compressed genomes as separate resources. Instead of storing the genome assemblies as separate data structures from the genome sequence data, the method and principles disclosed in this invention permit to store only the genome sequence data with associated metadata enabling reconstruction of the genomes used for alignment.

**Compressed representation of genomic sequence reads and reference sequences**

When sequence reads have been mapped with respect to a pre-existing or to a constructed reference sequence, each sequence read can be fully represented by a number of elements denoted in this disclosure as "genomic descriptors" or simply "descriptors".

For example, in the case of a sequence read that perfectly matches a segment of a reference sequence, the only sub-set of descriptors needed to represent the sequence read is composed by the coordinate of the mapping position on the reference (usually the coordinate of the mapping position of the left-most base of the sequence read), the length of the sequence read itself and the information indicating if the read is mapping on the direct or reverse DNA strand with respect to the reference sequence strand.

In case it is not possible to find any mapping position for which all the bases of the sequence read match all bases of the reference sequence, the mapping (or mappings) with the minimal number of mismatches are retained. In such case a different sub-set of descriptors is needed to also express the substitutions, the insertions, the deletions and the clipped bases that may occur in correspondence of the mapping position with the minimal or close to minimal number of mismatches. With such sub-set of descriptors the sequence reads can be reconstructed using the information carried by the descriptors and the information carried by the reference sequence.

The genome sequencing process may generate read duplicates (i.e. two or more exact copies of the same genomic sequence) for two main physical reasons:

- the occurrence of a Polymerase Chain Reaction duplicate,
- the occurrence of an optical duplicate in the data acquisition process. A read is called as an optical duplicate if the pair of reads are both on the same tile, and the distance between reads is less than a given configuration parameter depending on the experiment.

The mapping process can also produce other types of information such as: multiple possible mapping positions and the related scores, the quality of mapping, the specification of spliced reads, the mapping on two different references (usually chromosomes) of reads belonging to a pair, features of the sequencing process (e.g. PCR or optical duplicate). All such information requires specific additional descriptors that extend each sub-sets that then is compressed by applying for each sub-set of descriptors appropriate entropy coding algorithms.

Therefore each read or read pair can be uniquely represented by a specific sub-set of descriptors according to the results of the mapping process.

**Classification of the sequence reads according to matching rules**

The sequence reads generated by sequencing machines are classified by the disclosed invention into six different "classes" according to the matching results of the alignment with respect to one or more "pre-existing" reference sequences.

When aligning a DNA sequence of nucleotides with respect to a reference sequence the following cases can be identified:

- A region in the reference sequence is found to match the sequence read without any error (i.e. perfect mapping). Such sequence of nucleotides is referenced to as "perfectly matching read" or denoted as "Class P".
- A region in the reference sequence is found to match the sequence read with a type and a number of mismatches determined only by the number of positions in which the sequencing machine generating the read was not able to call any base (or nucleotide). Such type of mismatches are denoted by an "N", the letter used to indicate an undefined nucleotide base. In this document this type of mismatch are referred to as "n type" mismatch. Such sequences belong to "Class N" reads. Once the read is classified to belong to "Class N" it is useful to limit the degree of matching inaccuracy to a given upper bound and set a boundary between what is considered a valid matching and what it is not. Therefore, the reads assigned to Class N are also constrained by setting a threshold (MAXN) that defines the maximum number of undefined bases (i.e. bases called as "N") that a read can contain. Such classification implicitly defines the required minimum matching accuracy (or maximum degree of mismatch) that all reads belonging to Class N share when referred to the corresponding reference sequence, which constitutes an useful criterion for applying selective data searches to the compressed data. By way of example and not limitation, some analysis applications may require that mapped reads only contain a maximum of 3 undetermined ("N") bases when mapped to a reference genome to be considered acceptable candidates for further analysis. With existing formats such as SAM/BAM a processing pipeline has to decompress the entire dataset and parse all the decompressed records to discard reads with more than 3 "N" symbols and keep only those with 3 or less than 3 "N" symbols before proceeding with further analysis. According to an aspect of the present principles, an encoding application is able to compress separately reads with 3 or less "N" symbols so that a processing pipeline can decode and use them without any additional processing or storage needs.

• A region in the reference sequence is found to match the sequence read with types and number of mismatches determined by the number of positions in which the sequencing machine generating the read was not able to call any nucleotide base, if present (i.e. "n type" mismatches), plus the number of mismatches in which a different base, than the one present in the reference, has been called. Such type of mismatch denoted as "substitution" is also called Single Nucleotide Variation (SNV) or Single Nucleotide Polymorphism (SNP). In this document this type of mismatch is also referred to as "s type" mismatch. The sequence read is then referenced to as "M mismatching reads" and assigned to "Class M". Like in the case of "Class N", also for all reads belonging to "Class M" it is useful to limit the degree of matching inaccuracy to a given upper bound, and set a boundary between what is considered a valid matching and what it is not. Therefore, the reads assigned to Class M are also constrained by defining a set of thresholds, one for the number "n" of mismatches of "n type" (MAXN) if present, and

another for the number of substitutions "s" (MAXS). A third constraint is a threshold defined by any function of both numbers "n" and "s", f(n,s). Such third constraint enables to generate classes with an upper bound of matching inaccuracy according to any meaningful selective access criterion. For instance, and not as a limitation, f(n,s) can be (n+s)1/2 or (n+s) or any linear or non-linear expression that sets a boundary to the maximum matching inaccuracy level that is admitted for a read belonging to "Class M". Such boundary constitutes a very powerful criterion to implement sophisticated selective data searches to the compressed data when analyzing sequence reads for various purposes. By way of example and not limitation, it enables selection of compressed genomic reads containing any possible combination of the number of "n type" mismatches and "s type" mismatches (substitutions) beyond the simple threshold applied to the one type or to the other. Existing solutions such as the SAM/BAM format do not natively support the selection of aligned sequence reads having a user-defined number of mismatches with respect to the reference genome. Selecting genomic sequence reads having at most a number "N" of substitutions with respect to a reference genome would require:

1. the decompression of the entire BAM file into the textual SAM file
2. parsing the decoded SAM using text parsers configured to select the desired reads

This approach would require very large storage space (SAM text is about 2.5 time larger than BAM) and long processing time in the order of several hours for a 30x coverage. According to an aspect of the present principles, genomic sequence reads presenting any user-defined number of mismatches can be compressed separately in order to be available for decompression without the need to decompress the entire dataset.

• A fourth class is constituted by sequencing reads presenting at least one mismatch of any type among "insertion", "deletion" (a.k.a. *indels*) and "clipped", plus, if present, any mismatches type belonging to class N or M. Such sequences are referred to as "I mismatching reads" and assigned to "Class I". Insertions are constituted by an additional sequence of one or more nucleotides not present in the reference, but present in the read sequence. In this document this type of mismatch is referred to as "i type" mismatch. In literature when the inserted sequence is at the edges of the sequence it is also referred to as "soft clipped" (i.e. the nucleotides are not matching the reference but are kept in the aligned reads contrarily to "hard clipped" nucleotides which are discarded). In this document this type of mismatch is referred to as "c type" mismatch. Deletion are "holes" (missing nucleotides) in the read with respect to the reference. In this document this type of mismatch is referred to as "d type" mismatch. Like in the case of classes "N" and "M" it is possible and appropriate to define a limit to the matching inaccuracy. The definition of the set of constraints for "Class I" is based on the same principles used for "Class M" and is reported in Table 1 in the last table lines. Beside a threshold for each type of mismatch admissible for Class I data, a further constraint is defined by a threshold determined by any function of the number of the mismatches "n", "s", "d", "i" and "c", w(n,s,d,i,c). Such additional constraint make possible to generate classes with an upper bound of matching inaccuracy according to any meaningful user defined selective access criterion. For instance, and not as a limitation, w(n,s,d,i,c) can be (n+s+d+i+c)1/5 or (n+s+d+i+c) or any linear or non-linear expression that sets a boundary to the maximum matching inaccuracy level that is admitted for a read belonging to "Class I". Such boundary constitutes a very useful criterion for applying the desired selective data searches to the compressed data when analyzing sequence reads for various purposes because it enables to set a further boundary to any possible combination of the number of mismatches admissible in "Class I" reads beyond the simple threshold applied to each type of admissible mismatch.

- A fifth class includes all reads that do not find any mapping considered valid (i.e not satisfying the set of matching rules defining an upper bound to the maximum matching inaccuracy as specified in Table 1) for each data class when referring to the reference sequence. Such sequences are said to be "Unmapped" when referring to the reference sequences and are classified as belonging to the "Class U".

### Classification of read pairs according to matching rules

The classification specified in the previous section concerns single sequence reads. In the case of sequencing technologies that generates read in pairs (i.e. Illumina Inc.) in which two reads are known to be separated by an unknown sequence of variable length, it is appropriate to consider the classification of the entire pair to a single data class. A read that is coupled with another is said to be its "mate".

If both paired reads belong to the same class the assignment to a class of the entire pair is obvious: the entire pair is assigned to the same class for any class (i.e. P, N, M, I, U). In the case the two reads belong to a different class, but none of them belongs to the "Class U", then the entire pair is assigned to the class with the highest priority defined according to the following expression:

$$P < N < M < I$$

in which "Class P" has the lowest priority and "Class I" has the highest priority.

In case only one of the reads belongs to "Class U" and its mate to any of the Classes P, N, M, I a sixth class is defined as "Class HM" which stands for "Half Mapped".

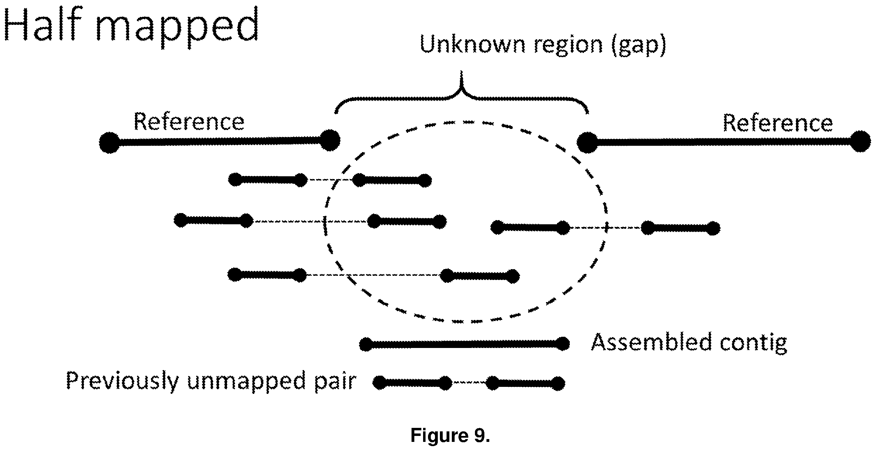

The definition of such specific class of reads is motivated by the fact that it is used for attempting to determine gaps or unknown regions existing in reference genomes (a.k.a. little known or unknown regions). Such regions are reconstructed by mapping pairs at the edges using the pair read that can be mapped on the known regions. The unmapped mate is then used to build the so called "contigs" of the unknown region as it is shown in figure 9. Therefore providing a selective access to only such type of read pairs greatly reduces the associated computation burden enabling much efficient processing of such data originated by large amounts of data sets than using the state of the art solutions would require to be entirely inspected.

The table below summarizes the matching rules applied to reads in order to define the class of data each read belongs to. The rules are defined in the first five columns of the table in terms of presence or absence of type of mismatches (n, s, d, i and c type mismatches). The sixth column provide rules in terms of maximum threshold for each mismatch type and any function f(n,s) and w(n,s,d,i,c) of the possible mismatch types.

**Table 2. Type of mismatches and set of constrains that each sequence reads must satisfy to be classified in the data classes defined in this invention disclosure.**

| Number and types of mismatches found when matching a read with a reference sequence | | | | | Set of matching accuracy constraints | Assignement Class |
|---|---|---|---|---|---|---|
| Number of unknown bases ("N") | Number of substitutions | Number of deletions | Number of Insertions | Number of clipped bases | | |
| 0 | 0 | 0 | 0 | 0 | 0 | P |
| n > 0 | 0 | 0 | 0 | 0 | n ≤ MAXN | N |
| | | | | | n > MAXN | U |

(continued)

| Number and types of mismatches found when matching a read with a reference sequence | | | | | Set of matching accuracy constraints | Assignement Class |
|---|---|---|---|---|---|---|
| Number of unknown bases ("N") | Number of substitutions | Number of deletions | Number of Insertions | Number of clipped bases | | |
| n ≥ 0 | s > 0 | 0 | 0 | 0 | n ≤ MAXN and s ≤ MAXS and f(n,s) ≤ MAXM | M |
| | | | | | n > MAXN or s > MAXS or f(n,s) > MAXM | U |
| n ≥ 0 | s ≥ 0 | d ≥ 0 * | i ≥ 0* | c ≥ 0* | n ≤ MAXN and s ≤ MAXS and d ≤ MAXD and i ≤ MAXI and c ≤ MAXC w(n,s,d,i,c) ≤ MAXTOT | I |
| | | **\*At least one mismatch of type d, i, c must be present (i.e. d>0 or i>0 or c>0)** | | | | |
| | | d ≥ 0 | i ≥ 0 | c ≥ 0 | n > MAXN or s > MAXS or d > MAXD or i > MAXI or c > MAXC w(n,s,d,i,c) > MAXTOT | U |

**Comparison with state of the art approaches**

Commonly used approaches such as SAM and CRAM do not encode reads or read pairs according to the specific sub-set of descriptors needed to represent their mapping information. SAM and CRAM do not classify sequence reads into data classes according to the number and type of mismatches they contain with respect to the reference sequence they are mapped to. Furthermore, these formats do not code sequence reads separately into Access Units containing in compressed form only sequence reads belonging to a single data class. In the case of sequence reads generated in pairs, state of the art approaches do not code them as single elements partitioned into classes according to their mapping accuracy with respect to the reference sequence. Such state of the art approaches are characterized by the following limitations and drawbacks:

1. The coding of reads or reads pairs without classifying sequence reads into separate data classes according to mapping results versus a reference sequence and using a unique super-set of descriptors is an inefficient approach that yields poor compression performance.
2. The lack of source modelling for the compressed data and the use of general purpose compressors such as ZIP, GZIP, LZMA produces poor compression ratios.
3. The coding of read pairs as separate sequence reads requires the duplication of several descriptors carrying the same information such as for example the read identifiers (a.k.a. read names), thus results inefficient and yields poor compression performance.
4. The retrieval of the information needed to reconstruct read pairs result to be complex and inefficient since the process requires a brute-force sequential search in possibly the entire dataset which can be extremely large in case of Next-Generation Sequencing (NGS) technology.
5. The selective access to read or read pairs mapped to specific genomic regions requires to search the entire dataset to have the guarantee that all reads or read pairs are retrieved.

When coding read pairs by means of a single sub-set of descriptors, the following technical advantages are evident to a person skilled in the art:

1. The information common to both reads, which is clearly redundant, is not replicated by coding a pair as single element (e.g. read pair identifiers, mapping distance, mapping reference identifiers, the various mapping quality information currently encoded by specific flags in the SAM file format)
2. The retrieval of the mutual pairing information (i.e. the information providing which read is the mate of any read at hand) is straightforward and does not require any further processing. Conversely in the state of the art approaches it may require to parse the entire volume of data.

In order to enable efficient selective access to specific portions of sequencing data and being able to transport them on a digital data network, the set of descriptors used to represent sequence reads aligned to a reference are structured in logically separate and independent data blocks called Access Units (AU). Each Access Unit contains only the compressed representation of a single data class, and can be decoded either independently of any other Access Units or using only Access Units carrying the coded representation of the reference sequence region used for mapping. This enables selective access and out-of-order transport capabilities.

In order to increase the compression efficiency this invention eliminates the need of specifying the "mapping reference identifier" descriptor for each read pair having both pairs mapped on the same reference sequence. Each Access Unit can contain only reads or pairs that map on the same reference. Using such solution the descriptor representing the reference sequence identifier needs to be encoded only once per each Access Unit or set of Access Units (and not repeated for each read as currently done in SAM/BAM formats).

The only exception of the rule expressed above is the case of read pairs having the two reads mapped on different reference sequences (e.g. chromosomes). In this case the pair is split and the two reads are coded as two separate genomic records and each encoded read contains the identifier of the reference sequence its mate is mapped to.

Experimental data, have demonstrated that developing entropy coders suited to the statistical properties of the genomic descriptors provide better compression performance with respect to the usage of a general purpose compressor (e.g. LZ type algorithm) applied to a heterogeneous set of data. As a consequence, when encoding genomic sequence reads in pairs by means of a specific sub-set of descriptors, higher compression is achieved thanks to the lower entropy characterizing each separate sub-set of descriptors and higher processing efficiency when reconstructing and retrieving read pairs.

The advantages in terms of achievable compression ratios provided by the approach disclosed in this invention are described in the next sections where the different binarizations and transformations applied to different blocks of genomic descriptors prior to entropy coding are described with the related performance.

### Encoding reference genomes with sequence data

In an embodiment the present principles are directed to the lossless compression of reference sequences such as reference genomes or genome assemblies when reference-less compression of aligned sequence data is performed. Reference-less compression of aligned sequence data according to Voges, J., Munderloh, M., Ostermann, J., "Predictive Coding of Aligned Next-Generation Sequencing Data" (2016 Data Compression Conference (DCC)) can be implemented using the genomic descriptors from 1 to 12 defined in Table 1 of this disclosure. Voges uses a circular buffer to progressively store encoded sequence reads and build the related contig by using the SAM CIGAR string associated to each aligned sequence. While this mentioned approach can achieve efficient compression of genome sequencing data 101 mapped on a reference sequence 100, it does not support the representation and compression of the reference sequence 100 itself, because a decoding engine will only be able to reconstruct the contig and the compressed genomic sequence reads, but the original reference genome used for the alignment is not contained in the compressed data. This disclosure aims at using the genomic descriptors no. 13 and 14 in Table 1 to achieve efficient compression of the reference genome used for alignment when reference-less compression is applied to the genome sequence data. This is achieved by storing in the compressed data the differences between the contigs assembled during the compression process and the reference genome used for alignment. At the decoding end, the decoding process will reconstruct the contig used for genomic sequence reads decompression and - by means of descriptors 13 and 14 - it will be able to reconstruct the reference genome used for alignment.

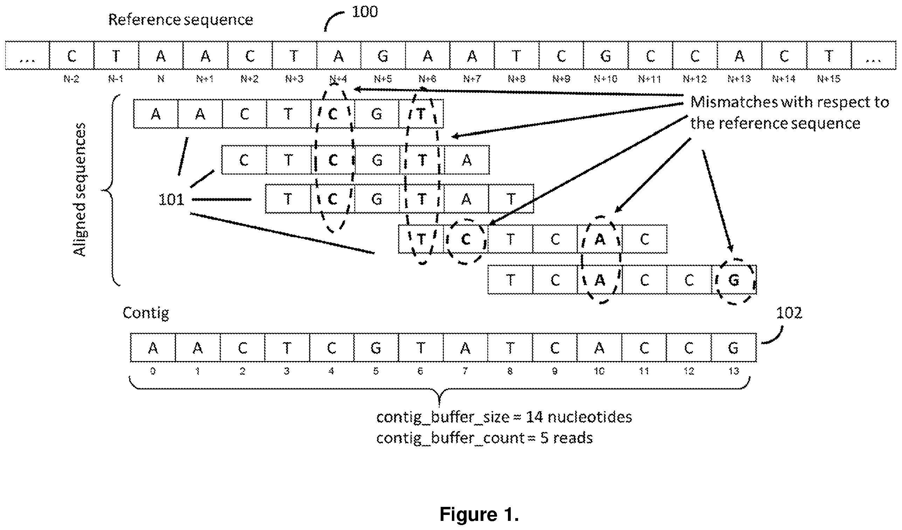

Figure 1 shows how aligned sequence reads 101 can be used to build a longer sequence 102 called contig to be used to perform reference-based compression. The contig is assembled by selecting, per each position on the reference genome, the nucleotide that is present with the highest frequency in the aligned reads at that position. If such nucleotide is the same as in the reference sequence this is said to be a "match" otherwise if it is different, it is said to be a "mismatch". This is not possible in the approach of Voges where the buffer has to be fixed a priori, also due to predefined design choices.

Moreover, in the present invention the length of the contig can be defined by the user (for example in a input parameter file) or dynamically updated by the encoder. The information about the length of the contig can be sent from the encoder to the decoder in a data structure contained in the file format used to store or transmit the compressed genomic information such as the one shown in Table 18 . Such new feature has the considerable advantage that it is defined by a parameter, it can be adapted to the various encoder and decoder architectures and their relevant limitations, and it is also adaptable to the evolution of the various architectures that will be used for encoding and decoding the genome sequences and to the computational complexity of the sequence to be coded.

The contig length can be expressed in terms of both the number of nucleotides and/or the number of reads used in the encoding and decoding process. This process is shown in figure

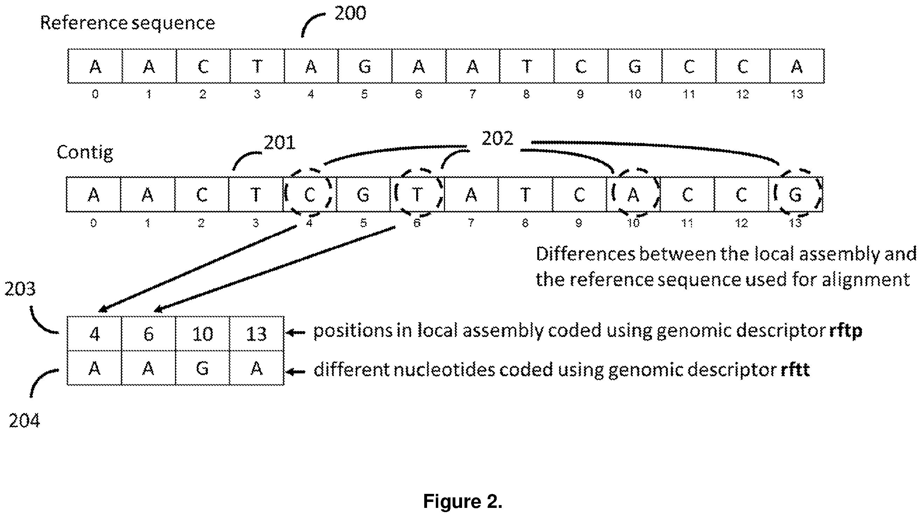

Additionally, figure 2 shows how said "mismatches" (202) between the reference sequence 200 and the contig 201 are coded using descriptors no. 13 and no. 14 of table 1. Entropy coding such descriptors and encapsulating them in the same Access Unit containing the descriptors used to compress the aligned sequence reads makes possible the reconstruction at the decoding device of the reference genome used for alignment.

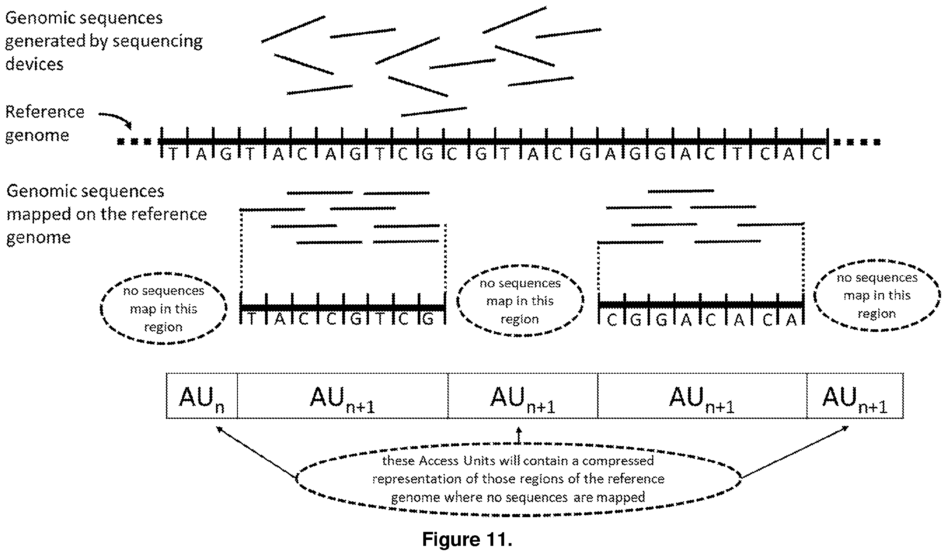

In another embodiment of the invention, regions of the reference genome used for the alignment that are not covered by any of the mapped reads, can be compressed (i.e. entropy coded), encapsulated and carried in specific Access Units. Such Access Units contain only the compressed representation of the reference genome used for the alignment and cover the genomic regions on which no sequence reads are mapped. This is shown in figure 11 where regions of the reference genome used for alignment, but on which no reads are mapped, are coded in specific Access Units. The technical advantage of such an invention is the possibility to completely reconstruct at the decoding end the reference genome used for the alignment without the need of storing an amount of data corresponding to the complete volume of a reference genome.

The technical advantages of such approach with respect to the solution of Voges et al. cited here are the following:

1. The reference genome used for alignment is available at the decoding device without any off-band transmission of additional data (e.g. the compressed genome or any other reference to external repositories)

2. The reference genome can be selectively reconstructed at the decoding device with a granularity equal to the genomic region covered by each Access Unit. Partial regions of the reference genome can be accessed with a random access mechanism. This enables much more efficient data processing with respect to existing solution which require decompression and manipulation of the entire 3.2 billion nucleotides composing a human reference genome even if the analysis is restricted to a much smaller genomic region

3. The **rftp** and **rftt** descriptors disclosed in this invention can be used by genome analysis pipelines as indicators of new Single Nucleotide Polymorphism (a.k.a. SNP, see https://en.wikipedia.org/wiki/Single-nucleotide_polymorphism) present in the compressed sequence data when the reference genome used for alignment belongs to the same individual the compressed sequence data belong to. It has to be appreciated that - at very high (more than 30x) coverage - the contig 102 built during the process of reference-less compression can be considered as a new assembly of an individual's genome. When the contig is compared to a previously obtained reference genome 100 belonging to the same individual, the found differences are an indication of the possible presence of a Single Nucleotide Polymorphism (a.k.a. SNP, see https://en.wikipedia.org/wiki/Single-nucleotide_polymorphism).

**Entropy coding of genomic descriptors**

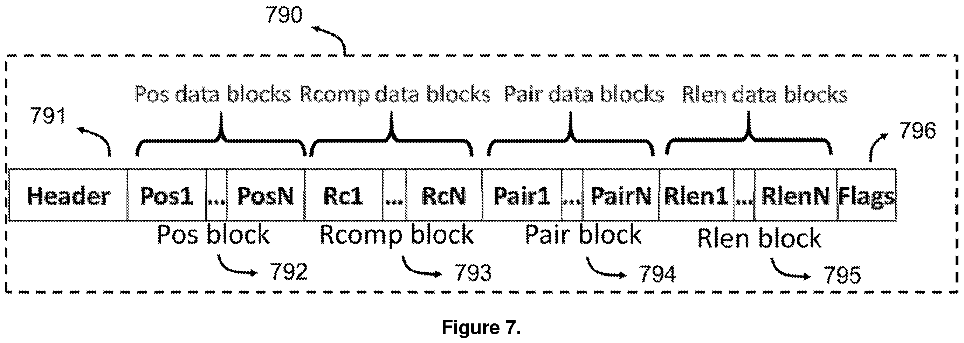

Sub-sets of genomic descriptors defined in this disclosure are used to represent genomic data belonging to the six classes defined according to the principle of this invention. Figures 6 and 7 show that genomic sequence reads mapped in contiguous regions of the reference sequences are represented by blocks of genomic descriptors encapsulated in one Access Unit. Said blocks of descriptors are entropy coded using different entropy coders specifically tailored to the statistical properties of each descriptor. This approach provides better compression ratios than other approaches such as SAM/BAM or CRAM because each block of genomic descriptors represent a source of information which can be modeled more efficiently than a SAM or CRAM record. SAM and CRAM records are groups of heterogeneous elements which do not share the same statistical properties as the genomic descriptors defined in this disclosure.

The transformations and binarizations applied to the descriptors together with the necessary configuration parameters for the entropy coders of said descriptors providing better compression ratios than state of the art solution and are disclosed in this invention as reported below.

*Transformation of descriptors*

The transformation of descriptors is a process whereby a descriptor's value representing a genomic feature such as a mismatch position within a genomic sequence or a mismatch type is transformed in a different corresponding value in order to achieve better compression performance. In an embodiment, according to the principles of this disclosure, the mismatches positions represented by the **rftp** descriptor are transformed according to the following steps:

1. The input to the transformation process are positions of mismatches expressed as distances in nucleotides from the first nucleotide of the sequence read. This is shown in figure 2 where the four mismatches 203 of the assembled contig with respect to the reference sequence are at position 4, 6, 10 and 13.

2. Each absolute position is then transformed into a differential position with respect to the previous mismatch. The first mismatch keeps the same value. The four positions values are then transformed in 4, 2, 4, 3. These values are the input to the binarization process of the **rftp** descriptor described below.

In an embodiment, according to the principles of this disclosure, the mismatches types represented by the **rftt** descriptor are transformed according to the following steps:

1. The input to the transformation process are type of mismatches expressed as nucleotides symbols. This is shown in figure 2 where the four mismatches 204 of the assembled contig with respect to the reference sequence are of type A, A, G, A.

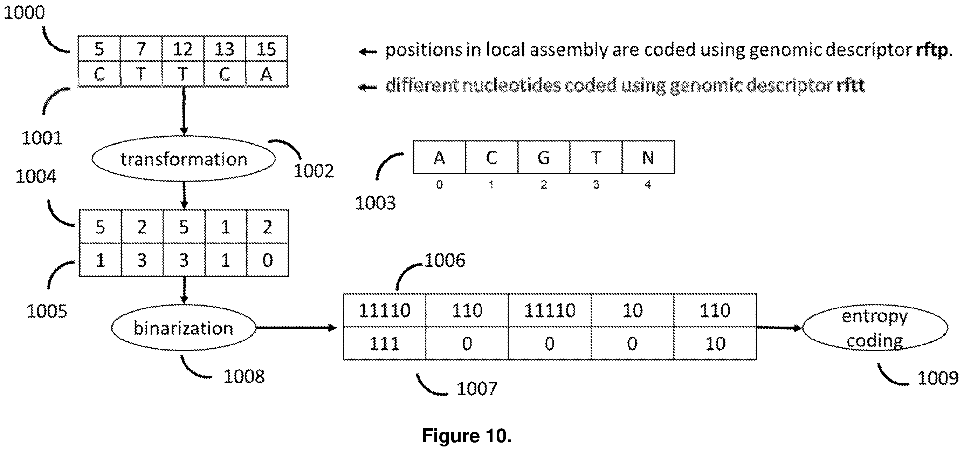

2. Each nucleotide is then transformed into an integer value representing the position of the nucleotide in a vector 209 containing all possible symbols. This is shown in figure 10. The four mismatches types are then transformed in 0, 0, 2, 0. These values are the input to the binarization process of the **rftt** descriptor described below.

## *Binarization of descriptors*

In an embodiment, the present invention uses context-adaptive binary arithmetic coding (CABAC) for the compression of the genomic descriptors. CABAC first converts to a binary representation all symbols to be encoded. The process of binarization converts a non-binary-valued symbol (e.g. a mapping position, a mapped read length or a mismatch type) into a binary code prior to arithmetic coding.

The selection of appropriate binarizations adapted to the statistical properties of each descriptor provides better compression ratios than existing formats based on general purpose compressor applied on blocks of heterogeneous elements.

In the following sections these variables are defined:

- *symVal* : non-binary value of the genomic descriptor to be binarized.
- *cLength*: represents the numbers of bits with which the value is binarized.
- *cMax*: is the largest possible value to be binarized. Larger values will be truncated.

While the following binarization tables are calculated for fixed values of these variables, it has to be appreciated that the present principles are not limited to these values, and thus other values can also be used in accordance with the present principles.

Each binarization algorithm used in this disclosure is identified by an identifier as shown in Table 3.

**Table 3. Type of binarizations and respective identifiers**

| binarization_id | Type of binarization |
|---|---|
| 0 | Binary Coding (BI) |
| 1 | Truncated Unary (TU) |
| 2 | Exponential Golomb (EG) |
| 3 | Signed Exponential Golomb (SEG) |
| 4 | Truncated Exponential Golomb (TEG) |
| 5 | Signed Truncated Exponential Golomb (STEG) |
| 6 | Split Unit-wise Truncated Unary (SUTU) |
| 7 | Signed Split Unit-wise Truncated Unary (SSUTU) |
| 8 | Double Truncated Unary (DTU) |
| 9 | Signed Double Truncated Unary (SDTU) |

### *Binary Coding (BI)*

This is a standard binary representation whereby each numerical value is coded in its binary representation. The variable cLength - shown in Table 15 when binarization id = 0 - represents the numbers of bits with which the value will be represented.

### *Truncated Unary (TU) Binarization*

A TU binary string is the concatenation of *symVal* ones followed by one zero. If *symVal* == *cMax*, the trailing 0-bit is discarded. Table 4 illustrates the bin strings of this truncated unary binarization with *cMax* = 3.

**Table 4. Bin string of the truncated unary binarization with cMax==3**

| symVal | Binary string | | |
|--------|---------------|---|---|
| 0 | 0 | | |
| 1 | 1 | 0 | |
| 2 | 1 | 1 | 0 |
| 3 | 1 | 1 | 1 |
| binIdx | 0 | 1 | 2 |

The syntax for this binarization process along with arithmetic decoding is described below.

```
decode_cabac_TU(ctxTable, ctxIdx, cMax) {
    for (binIdx=0; binIdx<cMax; binIdx++) {
        binValue
        if (binValue == 0)
            break
    }
    return binIdx
    }
```

**binValue** is the binarized value which can be either 0 or 1.

### Exponential Golomb (EG) Binarization

The parsing process for genomic descriptors binarized using this technique begins with reading the bits starting at the current location in the bitstream up to and including the first non-zero bit, and counting the number of leading bits that are equal to 0.

This process is specified as follows:

```
leadingZeroBits= -1
for( b = 0; !b; leadingZeroBits++ )      b = read_bits( 1 )
```

The variable *symVal* is then assigned as follows:

$$symVal = 2^{leadingZeroBits} - 1 + read\_bits( leadingZeroBits )$$

where the function call read bits reads a number of bits from a storage medium equal to the parameter passed as input. The value returned from read_bits( leadingZeroBits) is interpreted as a binary representation of an unsigned integer with the most significant bit written first.

Table 5 illustrates the structure of the Exp-Golomb code by separating the bit string into "prefix" and "suffix" bits. The "prefix" bits are those bits that are parsed as specified above for the computation of leadingZeroBits, and are shown as either 0 or 1 in the bit string column of Table 5. The "suffix" bits are those bits that are parsed in the computation of symVal and are shown as $x_i$ in Table 5, with i in the range of 0 to leadingZeroBits - 1, inclusive. Each $x_i$ is equal to either 0 or 1.

**Table 5. Binary representations for values of symVal from 0 to 62**

| Bit string form | Range of symVal |
|-----------------|-----------------|
| 1 | 0 |
| 0 1 $x_0$ | 1..2 |
| 0 0 1 $x_1$ $x_0$ | 3..6 |
| 0 0 0 1 $x_2$ $x_1$ $x_0$ | 7..14 |

(continued)

| Bit string form | Range of symVal |
|---|---|
| 0 0 0 0 1 $x_3$ $x_2$ $x_1$ $x_0$ | 15..30 |
| 0 0 0 0 0 1 $x_4$ $x_3$ $x_2$ $x_1$ $x_0$ | 31..62 |
| ... | ... |

Table 6 illustrates the explicit assignments of bit strings to symVal values.

**Table 6. Exp-Golomb bit strings and symVal in explicit form**

| Bit string | symVal |
|---|---|
| 1 | 0 |
| 0 1 0 | 1 |
| 0 1 1 | 2 |
| 0 0 1 0 0 | 3 |
| 0 0 1 0 1 | 4 |
| 0 0 1 1 0 | 5 |
| 0 0 1 1 1 | 6 |
| 0 0 0 1 0 0 0 | 7 |
| 0 0 0 1 0 0 1 | 8 |
| 0 0 0 1 0 1 0 | 9 |
| ... | ... |

Depending on the genomic descriptor, the value of a binarized syntax element is decoded using one of the following methods:

1. The value of the decoded genomic descriptor is equal to the *symVal* value corresponding to the binarized descriptor
2. The value of the decoded genomic descriptor is calculated by applying signed 0-order Exponential-Golomb decoding as defined for example in https://en.wikipedia.org/wiki/Exponential-Golomb coding with *symVal* as input.

### Signed Exponential Golomb (SEG) Binarization

According to this binarization method the genomic descriptor is associated to the *symVal* by ordering the syntax element by its absolute value in increasing order and representing the positive value for a given absolute value with the lower *symVal*. Table 7 shows the assignment rule.

**Table 7. Assignment of syntax element to symVal for signed Exp-Golomb coded genomic descriptors.**

| symVal | syntax element value |
|---|---|
| 0 | 0 |
| 1 | 1 |
| 2 | -1 |
| 3 | 2 |
| 4 | -2 |
| 5 | 3 |
| 6 | -3 |
| k | $(-1)^{k+1}$ Ceil( k ÷ 2 ) |

*Truncated Exponential Golomb (TEG) Binarization*

This binarization process requires the use of an additional input parameter *tegParam* which defines how the binarization is calculated.

Output of this process is the TEG binarization of the syntax element.

A TEG bin string is the concatenation of 1 (in case of symVal == 0) or 2 (in case of symVal > 0) types of binarization:

    1. The truncated unary binarization with cMax = tegParam for the value Min(symVal, tegParam)

    2. If symVal !=0, the exponential golomb binarization for the value Abs(symVal) - tegParam

Table 8 illustrates the bin strings of this Truncated Exponential Golomb binarization with tegParam ==2.

**Table 8. Bin string of the Truncated Exponential Golomb binarization with tegParam=2**

| symVal | Truncated Unary | | Exponential Golomb | | |
|---|---|---|---|---|---|
| 0 | 0 | - | - | - | - |
| 1 | 1 | 0 | - | - | - |
| 2 | 1 | 1 | 1 | - | - |
| 3 | 1 | 1 | 0 | 1 | 0 |
| 4 | 1 | 1 | 0 | 1 | 1 |
| ... | | | | | |
| binIdx | 0 | 1 | 2 | 3 | 4 |

*Signed Truncated Exponential Golomb (STEG) Binarization*

This binarization process requires the use of an additional input parameter *stegParam*.

A STEG binary string is the concatenation of 1 (in case of symVal == 0) or 2 (for other cases) binarizations:

    1. The truncated exponential golomb binarization for Abs(symVal)
    2. If symVal !=0, a one-bit flag equal to 1 (if symVal<0) or equal to 0 (if symVal>0).

Table 9 illustrates the bin strings of this Signed Truncated Exponential Golomb binarization with stegParam = 2.

**Table 9. Binary string of the Signed Truncated Exponential Golomb binarization with stegParam = 2**

| symVal | Truncated Exponential Golomb | | | | | Sign Flag |
|---|---|---|---|---|---|---|
| | Truncated Unary | | Exponential Golomb | | | |
| ... | | | | | | |
| -4 | 1 | 1 | 0 | 1 | 1 | 1 |
| -3 | 1 | 1 | 0 | 1 | 0 | 1 |
| -2 | 1 | 1 | 1 | - | - | 1 |
| -1 | 1 | 0 | - | - | - | 1 |
| 0 | 0 | - | - | - | - | - |
| 1 | 1 | 0 | - | - | - | 0 |
| 2 | 1 | 1 | 1 | - | - | 0 |
| 3 | 1 | 1 | 0 | 1 | 0 | 0 |
| 4 | 1 | 1 | 0 | 1 | 1 | 0 |
| ... | | | | | | |

(continued)

| symVal | Truncated Exponential Golomb | | | | | Sign Flag |
| --- | --- | --- | --- | --- | --- | --- |
| | Truncated Unary | | Exponential Golomb | | | |
| binIdx | 0 | 1 | 2 | 3 | 4 | Max(binIdx)+1 |

### Split Unit-wise Truncated Unary (SUTU) Binarization

This binarization process requires the use of two input parameters *splitUnitSize* and *outputSymSize*. *outputSymSize* must always be a multiple of *splitUnitSize*.

The SUTU binary string is a concatenation of repeated TU binarizations, where each TU binarization is applied to portions of *symVal* which are *splitUnitSize* bits long. In other words, *symVal* is represented by *x* binary string obtained with the TU binarization, where x = outputSymSize/splitUnitSize. The cMax parameter for each binary string is defined as cMax = (1<<splitUnitSize) - 1.

Table 10 illustrates the binary strings of split unit-wise truncated unary binarizations with splitUnitSize = 2 and outputSymbSize = 8.

**Table 10. Bin string of the Split Unit-wise Truncated Unary binarization with splitUnitSize = 2, outputSymSize = 8.**

| symVal | TU Instance 1 cMax==3 | | | TU Instance 2 cMax==3 | | | TU Instance 3 cMax==3 | | | TU Instance 4 cMax==3 | | |
| --- | --- | --- | --- | --- | --- | --- | --- | --- | --- | --- | --- | --- |
| 0 | 0 | - | - | 0 | - | - | 0 | - | - | 0 | - | - |
| 1 | 1 | 0 | - | 0 | - | - | 0 | - | - | 0 | - | - |
| 3 | 1 | 1 | 1 | 0 | - | - | 0 | - | - | 0 | - | - |
| 15 | 1 | 1 | 1 | 1 | 1 | 1 | 0 | - | - | 0 | - | - |
| 31 | 1 | 1 | 1 | 1 | 1 | 1 | 1 | 0 | - | 0 | - | - |
| 63 | 1 | 1 | 1 | 1 | 1 | 1 | 1 | 1 | 1 | 0 | - | - |
| binIdx | 0 | 1 | 2 | 3 | 4 | 5 | 6 | 7 | 8 | 9 | 10 | 11 |

The bitstream syntax for this binarization process is described below.

```
decode_cabac_SUTU(ctxTable, ctxIdx, splitUnitSize, outputSymSize) {
    output_symb = 0
    cMax = (1<<splitUnitSize) - 1
    for (i=0; i<outputSymSize; i+=splitUnitSize) {
        tmp = decode_cabac_TU(ctxTable, ctxIdx, cMax)
        ctxIdx += cMax
        output_sym |= (tmp<<i)
    }
    return output_sym
    }
```

**Table 11. CABAC decoding process for TU binarization.**

### Signed Split Unit-wise Truncated Unary (SSUTU) Binarization

This binarization process requires the use of two input parameters *splitUnitSize* and *outputSymSize*.

The SSUTU binary string is obtained by an extension of the SUTU binarization process with the sign of *symVal* coded as a separate flag.

- The SUTU binarization for the value Abs(symVal).
- If symVal !=0, a one-bit flag equal to 1 (if symVal<0) or equal to 0 (if symVal>0).

Table 12 illustrates the binary strings of the Signed Split Unit-wise Truncated Unary binarization with splitUnitSize = 2, outputSymbSize = 8.

**Table 12 Bin string of the Signed Split Unit-wise Truncated Unary binarization with splitUnitSize = 2, outputSymSize = 8.**

| symVal | TU Instance 1 cMax==3 | | | TU Instance 2 cMax==3 | | | TU Instance 3 cMax==3 | | | TU Instance 4 cMax==3 | | | Sign |
|---|---|---|---|---|---|---|---|---|---|---|---|---|---|
| -63 | 1 | 1 | 1 | 1 | 1 | 1 | 1 | 1 | 1 | 0 | - | - | 1 |
| -31 | 1 | 1 | 1 | 1 | 1 | 1 | 1 | 0 | - | 0 | - | - | 1 |
| -15 | 1 | 1 | 1 | 1 | 1 | 1 | 0 | - | - | 0 | - | - | 1 |
| -3 | 1 | 1 | 1 | 0 | - | - | 0 | - | - | 0 | - | - | 1 |
| -1 | 1 | 0 | - | 0 | - | - | 0 | - | - | 0 | - | - | 1 |
| 0 | 0 | - | - | 0 | - | - | 0 | - | - | 0 | - | - | - |
| 1 | 1 | 0 | - | 0 | - | - | 0 | - | - | 0 | - | - | 0 |
| 3 | 1 | 1 | 1 | 0 | - | - | 0 | - | - | 0 | - | - | 0 |
| 15 | 1 | 1 | 1 | 1 | 1 | 1 | 0 | - | - | 0 | - | - | 0 |
| 31 | 1 | 1 | 1 | 1 | 1 | 1 | 1 | 0 | - | 0 | - | - | 0 |
| 63 | 1 | 1 | 1 | 1 | 1 | 1 | 1 | 1 | 1 | 0 | - | - | 0 |
| binIdx | 0 | 1 | 2 | 3 | 4 | 5 | 6 | 7 | 8 | 9 | 10 | 11 | 12 |

The syntax for this binarization process is described below.

```
decode_cabac_SSUTU(ctxTable, ctxIdx, splitUnitSize, outputSymSize) {
    output_sym = decode_cabac_SUTU(ctxTable, ctxldx, splitUnitSize,
            outputSymSize)
    if(output_sym > 0) {
        ctxldx += ((1<<splitUnitSize) - 1) * (outputSymSize / splitUnitSize)
    sign_flag
    if(sign_flag == 1)
        output_sym = -output_sym
    }
    return output_sym
    }
```

**sign_flag** represents the cabac decoding of a bit on context variable identified by ctxIdx. **decode_cabac_SUTU()** represents the cabac decoding process for the SUTU binarization.

### Double Truncated Unary (DTU) Binarization

This binarization process requires the use of two input parameters *splitUnitSize* and *outputSymSize.*
The DTU binary string is a concatenation of two binarizations, namely the TU binarization and the SUTU binarization. The parameter *cMax* is used for the TU binarization, and parameters splitUnitSize and outputSymSize are used for the SUTU binarization (where its *cMax* is derived internally).

- The first instance of the TU binarization for the value Min(Abs(symVal), cMax).
- If Abs(symVal) > cMax, the second instance of SUTU binarization for the value Abs(symVal) - cMax.

Table 13 illustrates the binary strings of the Double Truncated Unary binarization with cMax = 1, splitUnitSize = 2, outputSymSize = 8.

**Table 13 Bin string of the Double Truncated Unary binarization with cMax = 1, splitUnitSize = 2, outputSymSize = 8.**

| symVal | TU Instance | SUTU Instance: splitUnitSize = 2, outputUnitSize = 8 | | | | | | | | | | | |
|--------|-------------|-------------------|---|---|-------------------|---|---|-------------------|---|---|-------------------|----|----|
| | | TU Instance 1 | | | TU Instance 2 | | | TU Instance 3 | | | TU Instance 4 | | |
| | cMax=1 | cMax=3 | | | cMax=3 | | | cMax=3 | | | cMax=3 | | |
| 0 | 0 | - | - | - | - | - | - | - | - | - | - | - | - |
| 1 | 1 | - | - | - | - | - | - | - | - | - | - | - | - |
| 3 | 1 | 1 | 1 | 0 | 0 | - | - | 0 | - | - | 0 | - | - |
| 15 | 1 | 1 | 1 | 0 | 1 | 1 | 1 | 0 | - | - | 0 | - | - |
| 31 | 1 | 1 | 1 | 0 | 1 | 1 | 1 | 1 | 0 | - | 0 | - | - |
| 63 | 1 | 1 | 1 | 0 | 1 | 1 | 1 | 1 | 1 | 1 | 0 | - | - |
| binIdx | 0 | 1 | 2 | 3 | 4 | 5 | 6 | 7 | 8 | 9 | 10 | 11 | 12 |

The binarization process is described below.

```
decode_cabac_DTU(ctxTable, ctxIdx, cMax, splitUnitSize, outputSymSize) {
  output_sym = 0
if(cMax > 0) {
     output_sym = decode_cabac_TU(ctxTable, ctxldx, cMax)
  if(output_sym > cMax) {
  output_sym += decode_cabac_SUTU(ctxTable, ctxldx+cMax,
        splitUnitSize, outputSymSize)
     }
  } else
  output_sym = decode_cabac_SUTU(ctxTable, ctxldx,
        splitUnitSize, outputSymSize)
return output_sym
}
```

**decode_cabac_TU()** represents the cabac decoding process for TU binarization.
**decode_cabac_SUTU()** represents the cabac decoding process for SUTU binarization.

### *Signed Double Truncated Unary (SDTU) Binarization*

This binarization process requires the use of two additional input parameters *splitUnitSize* and *outputSymSize.*
The SDTU binary string is obtained by an extension of the DTU binarization process with the sign of *symVal* coded as a flag.

- The DTU binarization for the value Abs(symVal).
- If symVal !=0, a one-bit flag equal to 1 (if symVal<0) or equal to 0 (if symVal>0).

Table 14 illustrates the bin strings of double truncated unary binarization with with cMax = 1, splitUnitSize = 2, outputSymSize = 8.

**Table 14 Bin string of the Signed Double Truncated Unary binarization with with cMax = 1, splitUnitSize = 2, outputSymSize = 8.**

| symVal | TU Instance | SUTU Instance: splitUnitSize = 2, outputUnitSize = 8 | | | | | | | | | | | | Sign |
|--------|-------------|-------------------|---|---|-------------------|---|---|-------------------|---|---|-------------------|---|---|------|
| | | TU Instance 1 | | | TU Instance 2 | | | TU Instance 3 | | | TU Instance 4 | | | |
| | cMax=1 | cMax=3 | | | cMax=3 | | | cMax=3 | | | cMax=3 | | | |
| -63 | 1 | 1 | 1 | 0 | 1 | 1 | 1 | 1 | 1 | 1 | 0 | - | - | 1 |
| -31 | 1 | 1 | 1 | 0 | 1 | 1 | 1 | 1 | 0 | - | 0 | - | - | 1 |

(continued)

| symVal | TU Instance cMax=1 | SUTU Instance: splitUnitSize = 2, outputUnitSize = 8 | | | | | | | | | | | | Sign |
|---|---|---|---|---|---|---|---|---|---|---|---|---|---|---|
| | | TU Instance 1 cMax=3 | | | TU Instance 2 cMax=3 | | | TU Instance 3 cMax=3 | | | TU Instance 4 cMax=3 | | | |
| -15 | 1 | 1 | 1 | 0 | 1 | 1 | 1 | 0 | - | - | 0 | - | - | 1 |
| -3 | 1 | 1 | 1 | 0 | 0 | - | - | 0 | - | - | 0 | - | - | 1 |
| -1 | 1 | - | - | - | - | - | - | - | - | - | - | - | - | 1 |
| 0 | 0 | - | - | - | - | - | - | - | - | - | - | - | - | - |
| 1 | 1 | - | - | - | - | - | - | - | - | - | - | - | - | 0 |
| 3 | 1 | 1 | 1 | 0 | 0 | - | - | 0 | - | - | 0 | - | - | 0 |
| 15 | 1 | 1 | 1 | 0 | 1 | 1 | 1 | 0 | - | - | 0 | - | - | 0 |
| 31 | 1 | 1 | 1 | 0 | 1 | 1 | 1 | 1 | 0 | - | 0 | - | - | 0 |
| 63 | 1 | 1 | 1 | 0 | 1 | 1 | 1 | 1 | 1 | 1 | 0 | - | - | 0 |
| binIdx | 0 | 1 | 2 | 3 | 4 | 5 | 6 | 7 | 8 | 9 | 10 | 11 | 12 | 13 |

The syntax for this binarization process is described below.

```
decode_cabac_SDTU(ctxTable, ctxIdx, cMax, splitUnitSize, outputSymSize) {
   output_sym = decode_cabac_DTU(ctxTable, ctxldx, cMax,
            splitUnitSize, outputSymSize)
   if(output_sym > 0) {
   ctxldx += cMax + ((1<<splitUnitSize) - 1) *
               (outputSymSize / splitUnitSize)
      sign_flag
   if(sign_flag == 1)
         output_sym = -output_sym
   }
 return output_sym
 }
```

**sign_flag** represents the cabac decoding of a bit on context variable identified by ctxIdx. **decode_cabac_DTU()** represents the cabac decoding with DTU binarization.

*Binarization parameters*

Each binarization algorithm introduced in the previous sections requires configuration parameters at the encoding and decoding ends. In an embodiment, said configuration parameters are encapsulated in a data structure described in Table 15. Each binarization algorithm is identified by an identifier as listed in Table 3.

**Table 15. Binarization parameters structure**

| Binarization ID | parameters |
|---|---|
| 0 | cLength |
| 1 | cMax |
| 2 | - |
| 3 | - |
| 4 | teg Param |
| 5 | steg Param |
| 6 | splitUnitSize, outputSymSize |

(continued)

| Binarization ID | parameters |
|---|---|
| 7 | splitUnitSize, outputSymSize |
| 8 | cMax, splitUnitSize, outputSymSize |
| 9 | cMaxsplitUnitSize, outputSymSize |

In Table 15 the following semantics applies:

**cMax** represents the largest value to be binarized. Larger values will be truncated.
**cLength** represents the numbers of bits with which the value is binarized.
**tegParam** represents the tegParam variable defined in this document for TEG binarization.
**stegParam** represents the stegParam variable defined in this document for STEG binarization.
**splitUnitSize** represents the splitUnitSize variable defined in this document for SUTU, SSUTU and DTU binarizations.
**outputSymSize** represents the outputSymSize variable defined in this document for SUTU, SSUTU DTU and SDTU binarizations.

**Evidence of the technical advantage of the present invention**

By applying the indicated CABAC binarization to the respective genomic descriptors as indicated in Table 16, the compression performance reported in * no additional information is necessary since it is already available in the compressed representation according to the principles of this disclosure Table 17 can be obtained. The improvement in compression performance of the method described in this disclosure can be appreciated by comparison with the corresponding file sizes of BAM and CRAM approaches and one of the best compressors in literature known as DeeZ (see Numanagic, I., et al "Comparison of high-throughput sequencing data compression tools", Nature Methods (ISSN: 1548-7091), vol. 13, p. 1005-1008 London: Nature Publishing Group, 2016). It has to be appreciated that the DeeZ, BAM and CRAM compression performance are calculated by adding the size of the compressed reference genome used for alignment to the sizes of the compressed genome sequence data. According to the principles of the present disclosure, the reference genome is embedded in the compressed file. In today practice said compressed reference genome is a FASTA (ASCII text) file compressed using general purpose compressors such as GZIP, LZMA, Bzip2. In the proposed comparison the reference genome hs37d5.fa was compressed using the **xz** Linux command with the option of maximum compression (-9).

**Table 16. Binarizations associated to each genomic descriptor**

| descriptor_id | binarization_id |
|---|---|
| 1 | 8 |
| 2 | 6 |
| 3 | 6 |
| 4 | 1 |
| 5 | 6 |
| 6 | 1 |
| 7 | concatenation of 5, 1, 3, 0 on the bytes composing each descriptor |
| 8 | 0 |
| 9 | 6 |
| 10 | 3 |
| 11 | 1 |
| 12 | 1 |
| 13 | 6 |

(continued)

| descriptor_id | binarization_id |
|---|---|
| 14 | 1 |

***Binarization applied to descriptors rftp and rftt***

An example of binarization of rftp and rftt is provided in this section and illustrated in figure 10.

The descriptors associated to five mismatches between a contig and a reference genome used for alignment are shown below:

| rftp | 5 | 7 | 12 | 13 | 15 |
|---|---|---|---|---|---|
| rftt | C | T | T | C | A |

Each nucleotide symbol is associated to an integer code:

| Nucleotide | code |
|---|---|
| A | 0 |
| C | 1 |
| G | 2 |
| T | 3 |
| N | 4 |

After transformation the values become:

| rftp | 5 | 2 | 5 | 1 | 2 |
|---|---|---|---|---|---|
| rftt | 1 | 3 | 3 | 1 | 0 |

The binarized values for **rftp** are calculates as follows:

1. The terminator value can be binarized as 0 or 1. Here for this example we select 0.
2. If terminator = 0 then binarization no. 6 with splitUnitSize = 4, outputSymbolSize = 12 is used and the following binary strings are associated to the values of rftp

a. 5 = 11110
b. 2 = 110
c. 5 = 11110
d. 1 = 10
e. 2 = 110

The binarized values for **rftt** are calculates as follows:
1. Knowing the nucleotide present in the reference genome, remove the corresponding symbol from the possible symbols to be encoded. I.e. for the first mismatch of the example, if the corresponding symbol in the reference is a 'G', the space of possible symbols to be encoded is 0, 1, 3, 4.
2. The frequencies of symbols of the mismatches types on the data to be encoded are measured and indexed from 0 to 3. Index 0 is affected to the most frequent mismatch and index 3 is affected to the less frequent mismatch. In this example an indexing could be: { 0 => 3, 1=> 0, 2=>4, 3=>1}
3. In the given example the five mismatches would be binarized using the TU binarization as:

| Symbol | index | TU binarization with cMax = 3 |
|---|---|---|
| 1 | 3 | 111 |

(continued)

| Symbol | index | TU binarization with cMax = 3 |
|---|---|---|
| 3 | 0 | 0 |
| 3 | 0 | 0 |
| 1 | 3 | 0 |
| 0 | 1 | 10 |

With the binarization approach shown above the following compression results are achieved:

**Table 17. Compression performance with respect to state of the art solutions (sizes in bytes).**

| Compressor | BAM | CRAM | Deez | Proposed method |
|---|---|---|---|---|
| 9827_2#49.bam (ERR317482) - Low coverage | 4,755,859,110 | 3,124,448,497 | 2,592,665,720 | 2,164,362,407 |
| Reference genome hs37d5.fa | 707,712,316 | 707,712,316 | 707,712,316 | N/A* |
| **Total** | **5,463,571,426** | **3,832,160,813** | **3,300,378,036** | **2,164,362,407** |
| NA12878_S1.bam - High Coverage | 117,653,446,187 | 64,565,636,391 | 64,334,196,408 | 47,759,141,388 |
| Reference genome hs37d5.fa | 707,712,316 | 707,712,316 | 707,712,316 | N/A* |
| **Total** | **118,361,158,503** | **65,273,348,707** | **65,041,908,724** | **47,759,141,388** |
| * no additional information is necessary since it is already available in the compressed representation according to the principles of this disclosure | | | | |

**Coding parameters**

In an embodiment, the parameters needed to encode and decode each Access Unit are encapsulated in a data structure named as defined in Table 18.

**Table 18. Coding parameters for genomic descriptors**

| Parameter name | Cardinality | Description |
|---|---|---|
| dataset type | 1 | type of data encoded in Access Units referring to this encoding parameters. |
| reads length | 1 | length in nucleotides of sequence reads in case of constant reads length. The value 0 indicates the presence of variable reads length (conveyed by the **rlen** syntax element as defined in this disclosure). |
| QV depth | 1 | number of Quality Values associated to each coded nucleotide. 0 means that no Quality Values are encoded. |
| alignment score depth | 1 | number of alignments scores associated to each coded alignments. 0 means that no alignment scores are encoded. |
| terminator size | 1 | represents the size in bytes minus one (e.g. 0 = 1 byte) of the terminator symbol used for the **mmpos** descriptor defined in Table 1 |
| terminator value | 1 | represents the value of the terminator symbol used for the **mmpos** descriptor defined in Table 1. |
| number of classes | 1 | number of data classes encoded in all Access Units referring to these encoding parameters. |
| class ID | number of classes | identifier associated to one of the data class defined in this disclosure (P, N, M, I, HM, U). |

(continued)

| Parameter name | Cardinality | Description |
|---|---|---|
| number of descriptors | 1 | total number of descriptors contained in Access Units referring to these configuration parameters |
| coding mode ID | number of descriptors | one of the coding modes defined in this disclosure |
| decoder configuration | number of descriptors | data structure containing the decoder configuration parameters as specified in this disclosure |
| number of groups | 1 | number of different values of the **rgroup** descriptor listed in table 1 present in all Access Units referring to the current encoding parameters. |
| group name | number of groups | null-terminated string identifier of a read group. |
| multiple alignments flag | 1 | flag signaling the presence of multiple alignments in the Access Unit. When set to 0 no multiple alignments are present |
| spliced reads flag | 1 | flag signaling the presence of spliced reads in the Access Unit. When set to 0 no spliced reads are present. |
| multiple signature base | 1 | flag signaling the use of multiple signatures in an Access Unit containing unmapped sequence reads (Class U). |
| signature size | 1 | size in bits of each integer representing an encoded signature. |
| score exponent | 1 | number of bits used to encode the exponent part of the multiple alignments score encoded in the **mscore** descriptor. As specified in IEEE RFC 754 the value can go from 0 to 11. The **mscore** descriptor is defined in table 1. |
| score fractional | 1 | number of bits used to encode the fractional part of the multiple alignments score encoded in the **mscore** descriptor. As specified in IEEE RFC 754 the value can go from 0 to 52. The **mscore** descriptor is defined in table 1. |
| contig buffer size | 1 | size in bytes of the buffer used to build the contig 102 in figure 1. |
| contig buffer count | 1 | number of reads used to build the contig 102 in figure 1. |

**Encoding apparatus**

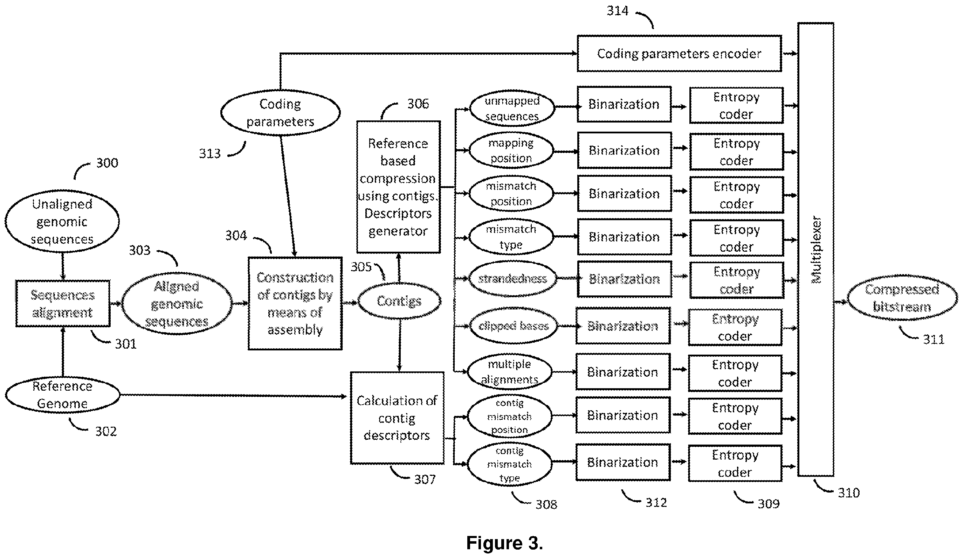

Figure 3 shows an encoding apparatus according to the principles of this invention. The encoding apparatus receives as input a reference genome 302 and unaligned genomic sequences 300, for example produced by a genome sequencing apparatus. Genome sequencing apparatus are known in the art, like the Illumina HiSeq 2500, the Thermo-Fisher Ion Torrent devices or the Oxford Nanopore MinION. The unaligned sequence data 300 are fed to a reads alignment unit 301, which maps the sequences on a reference genome 302. The aligned genomic sequences 303 are then used by an assembly apparatus 304 to build one or more contigs 305. The construction of contigs can be configured by coding parameters 313 such as the contig length or the number of sequence reads used to build each contig. The constructed contigs 305 are then used to perform reference based compression on the aligned genomic sequences 303. The reference based compressor 306 generates syntax elements named descriptors representing both mapped and unmapped genomic sequences. The reference genome 302 used for alignment and the constructed contigs 305 are fed to a reference genome differential coding apparatus 307 which generates descriptors representing the positions and type of mismatches between the reference genome 302 and the contigs 305. The genomic descriptors 308 generated by the reference based compressor 306 and the reference genome differential coder 307 are first binarized by several binarization units 312 and then entropy coded by several entropy coders 309. The entropy coded genomic descriptors are then fed to a multiplexing apparatus 310 to build one or more Access Unit composing a compressed bitstream 311. The multiplexed bitstream contains as well coding parameters structures 313 built by a coding parameters encoder 314.

Each Access Unit contains entropy coded descriptors representing alignment information and sequence reads belonging to one class of data as defined in this disclosure.

**Decoding apparatus**

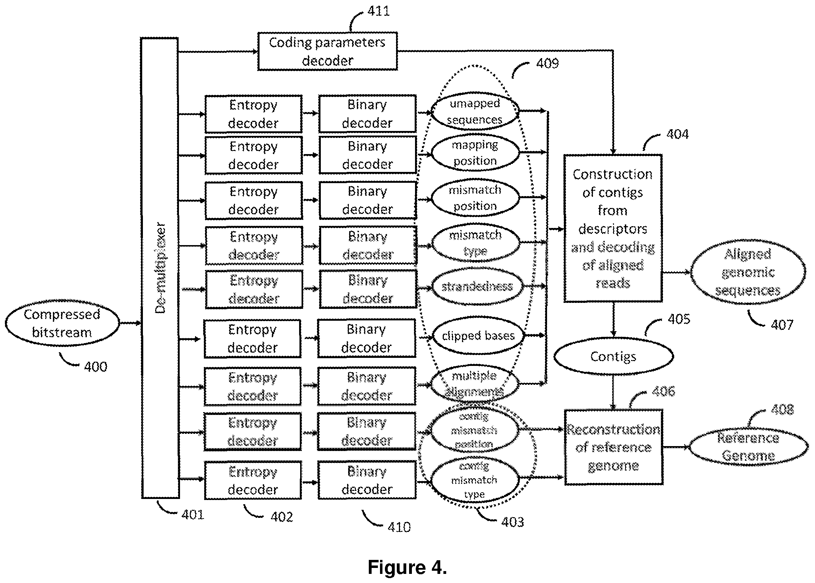

Figure 4 shows a decoding apparatus according to the principles of this disclosure. A demultiplexing unit 401 receives a multiplexed bitstream 400 from a network or a storage element and extracts the entropy coded payload of the Access Units composing said bitstream. Entropy decoders 402 receive the extracted payloads and decode the different types of genomic descriptors into their binary representations. Said binary representations are then fed to several binary decoders 410 which generate genomic descriptors 403 and 409. A coding parameter decoder 411 receives coding parameters multiplexed with the genomic information and feds them to the unit 404 in charge of constructing the contigs for sequence reads decoding. Genomic descriptors representing genomic sequence reads 409 are fed to a sequence reads reconstruction unit 404 which builds one or more contigs 405 as part of the decoding process and reconstructs the aligned genomic sequences 407. The contigs 405 and the entropy decoded descriptors 403 representing the differences between the contigs and the reference genome used for alignment are then fed to a reference genome reconstruction unit 406 which reconstructs the reference genome 408 used for alignment.

The inventive techniques herewith disclosed may be implemented in hardware, software, firmware or any combination thereof. When implemented in software, these may be stored on a computer medium and executed by a hardware processing unit. The hardware processing unit may comprise one or more processors, digital signal processors, general purpose microprocessors, application specific integrated circuits or other discrete logic circuitry.

The techniques of this disclosure may be implemented in a variety of devices or apparatuses, including mobile phones, desktop computers, servers, tablets and similar devices.

**Claims**

1. A computer-implemented method for encoding aligned genome sequence data comprising reads of sequences of nucleotides, said method comprising the steps of:

   • aligning said reads to one or more reference sequences thereby creating aligned reads,
   • mapping, on said one or more reference sequences, said aligned reads to be coded,
   • assembling said aligned reads thereby creating a contig,
   • comparing said one or more reference sequences and said contig thereby obtaining information related to the position of mismatches and the type of mismatches between the one or more reference sequences and the contig,
   • entropy coding said information related to the position of mismatches and the type of mismatches, and
   • wherein said information related to position of mismatches and the type of mismatches are indicated using, respectively a first descriptor (203) and a second descriptor (204), and said first descriptor and second descriptor are encapsulated in a same Access Unit so to enable, at the decoding device, the selective reconstruction of the one or more reference sequences used for the alignment.

2. The method of claim 1, wherein assembling said aligned reads comprises the step of selecting, for each position on the one or more reference sequences, the nucleotide that is present with the highest frequency in the aligned reads at that position.

3. The method of claim 1 or 2, wherein the length of said contig is defined as input parameter to the encoder or dynamically adapted by the encoder, preferably said first descriptor is binarized using a Split Unit-wise Truncated Unary binarization, wherein said Split Unit-wise Truncated Unary is a concatenation of repeated truncated unary binarizations, where each truncated unary binarization is applied to portions of the value to be binarized which are N bits long, wherein N is a preselected parameter, wherein said length of said contig is contained in a syntax header.

4. The method of claim 3, wherein the length of said contig is contained in a syntax header and said first descriptor is binarized using a Split Unit-wise Truncated Unary binarization, wherein said Split Unit-wise Truncated Unary is a concatenation of repeated truncated unary binarizations, where each truncated unary binarization is applied to portions of the value to be binarized which are N bits long, wherein N is a preselected parameter, and wherein said second descriptor is binarized using a Truncated Unary binarization, wherein the value of said second descriptor is followed by a zero and if said value is equal to the largest possible value to be binarized the trailing 0-bit is discarded.

5. The method of claim 3, wherein said method does not encode information signaling the usage of a specific reference

# EP 3 583 249 B1

sequence.

6. An apparatus for encoding genome sequence data, said genome sequence data comprising reads of sequences of nucleotides, said apparatus comprising means for:

- aligning said reads to one or more reference sequences thereby creating aligned reads,
- mapping, on said one or more reference sequences, said aligned reads to be coded,
- assembling said aligned reads thereby creating a contig,
- comparing said one or more reference sequences and said contig thereby obtaining information related to the position of mismatches and the type of mismatches between the one or more reference sequences and the contig,
- entropy coding said information related to the position of mismatches and the type of mismatches, and
- wherein said information related to position of mismatches and the type of mismatches are indicated using, respectively a first descriptor (203) and a second descriptor (204), and said first descriptor and second descriptor are encapsulated in the same Access Unit so to enable, at the decoding device, the selective reconstruction of the one or more reference sequences used for the alignment.

7. The apparatus of claim 6, further comprising means for executing the encoding method of any of Claims 2 to 5.

8. A computer-implemented method for decoding encoded genome sequence data, comprising the steps of:

parsing the encoded input file, so to obtain access units of genomic data,
entropy decoding information related to position of a mismatch and type of mismatch in a contig, and
modifying the contig by employing said information related to positions and types of mismatches thereby obtaining one or more reference sequences used for the alignment before compression, wherein modifying the contig by employing said information related to positions and types of mismatches thereby obtaining said one or more reference sequences further comprises entropy decoding of a first descriptor (203) and a second descriptor (204), and further comprising decapsulating from the same Access Unit said first descriptor and second descriptor so to obtain the selective reconstruction of said one or more reference sequences.

9. The method of claim 8, further comprising decoding the length of said contig from a syntax header contained in the input file.

10. The method of claim 8, further comprising a reverse binarization of said first descriptor, wherein said first descriptor is binarized using a Split Unit-wise Truncated Unary binarization, wherein said Split Unit-wise Truncated Unary is a concatenation of repeated truncated unary binarizations, where each truncated unary binarization is applied to portions of the value to be binarized which are N bits long, wherein N is a preselected parameter, preferably further comprising a reverse binarization of said second descriptor wherein said second descriptor is binarized using a Truncated Unary binarization, wherein the value of said second descriptor is followed by a zero and if said value is equal to the largest possible value to be binarized the trailing 0-bit is discarded.

11. The method of claim 8, wherein said input file does not contain information signaling the usage of a specific reference sequence.

12. An apparatus for decoding encoded genome sequence data, comprising means for:

parsing the encoded input file, so to obtain access units of genomic data,
entropy decoding information related to position of a mismatch and type of mismatch in a contig, and
modifying the contig by employing said information related to positions and types of mismatches thereby obtaining one or more reference sequences used for the alignment before compression, wherein modifying the contig by employing said information related to positions and types of mismatches thereby obtaining said one or more reference sequences further comprises entropy decoding of a first descriptor (203) and a second descriptor (204), and further comprising decapsulating from the same Access Unit said first descriptor and second descriptor so to obtain the selective reconstruction of said one or more reference sequences.

13. The apparatus of claim 12, further comprising means for executing the decoding method of any of claims 9 to 11.

14. A computer-readable medium storing instructions which, when executed by one or more processors, cause said one or more processors to carry out the method of any of claims 1-5.

**15.** A computer-readable medium storing instructions which, when executed by one or more processors, cause said one or more processors to carry out the method of any of claims 8-11.

**Patentansprüche**

**1.** Computerimplementiertes Verfahren zum Codieren von alignierten Genomsequenz-Daten, die Reads von Sequenzen von Nukleotiden umfassen, wobei das Verfahren die Schritte umfasst:

- Alignieren der Reads mit einer oder mehreren Referenzsequenzen, wodurch alignierte Reads geschaffen werden,
- Abbilden der alignierten Reads, die codiert werden sollen, auf eine oder mehrere Referenzsequenzen,
- Assemblieren der alignierten Reads, wodurch ein Contig erzeugt wird,
- Vergleichen der einen oder mehreren Referenzsequenzen und des Contigs, wodurch Informationen bezüglich der Position von Übereinstimmungsfehlern und des Typs von Übereinstimmungsfehlern zwischen der einen oder mehreren Referenzsequenzen und dem Contig erhalten werden,
- Entropie-Codieren der Informationen bezüglich der Position von Übereinstimmungsfehlern und des Typs von Übereinstimmungsfehlern, und
- wobei die Informationen bezüglich der Position von Übereinstimmungsfehlern und des Typs von Übereinstimmungsfehlern unter Verwendung von jeweils einem ersten Deskriptor (203) und einem zweiten Deskriptor (204) angegeben werden, und der erste Deskriptor und der zweite Deskriptor in einer selben Zugriffseinheit eingekapselt sind, um so an der Decodierungsvorrichtung die selektive Rekonstruktion der einen oder der mehreren für das Alignment verwendeten Referenzsequenzen zu ermöglichen.

**2.** Verfahren nach Anspruch 1, wobei das Assemblieren der alignierten Reads das Auswählen, für jede Position auf der einen oder den mehreren Referenzsequenzen, jenes Nukleotids umfasst, das mit der höchsten Frequenz in den alignierten Reads an der Position vorkommt.

**3.** Verfahren nach Anspruch 1 oder 2, wobei die Länge des Contigs als Eingabeparameter für den Codierer definiert ist oder dynamisch von dem Codierer adaptiert wird, wobei der erste Deskriptor vorzugsweise unter Verwendung einer geteilten, einheitsweise abgeschnittenen unären Binarisierung binarisiert wird, wobei die geteilte, einheitsweise abgeschnittene unäre Binarisierung eine Verkettung von wiederholten abgeschnittenen unären Binarisierungen ist, bei welcher jede abgeschnittene unäre Binarisierung auf Abschnitte des zu binarisierenden Werts angewandt wird, die N Bits lang sind, wobei N ein vorgewählter Parameter ist, wobei die Länge des Contigs in einem Syntax-Header enthalten ist.

**4.** Verfahren nach Anspruch 3, wobei die Länge des Contigs in einem Syntax-Header enthalten ist und der erste Deskriptor unter Verwendung einer geteilten, einheitsweise abgeschnittenen unären Binarisierung binarisiert wird, wobei die geteilte, einheitsweise abgeschnittene unäre Binarisierung eine Verkettung von wiederholten abgeschnittenen unären Binarisierungen ist, bei welcher jede abgeschnittene unäre Binarisierung auf Abschnitte des zu binarisierenden Werts angewandt wird, die N Bits lang sind, wobei N ein vorgewählter Parameter ist, und wobei der zweite Deskriptor unter Verwendung einer abgeschnittenen unären Binarisierung binarisiert wird, wobei auf den Wert des zweiten Deskriptors eine Null folgt, und wenn der Wert gleich dem höchsten möglichen zu binarisierenden Wert ist, das nachgestellte 0-Bit verworfen wird.

**5.** Verfahren nach Anspruch 3, wobei das Verfahren keine Informationen codiert, welche die Verwendung einer spezifischen Referenzsequenz signalisieren.

**6.** Vorrichtung zum Codieren von Genomsequenz-Daten, wobei die Genomsequenz-Daten Reads von Sequenzen von Nukleotiden umfassen, wobei die Vorrichtung Mittel umfasst zum:

- Alignieren der Reads mit einer oder mehreren Referenzsequenzen, wodurch alignierte Reads geschaffen werden,
- Abbilden der alignierten Reads, die codiert werden sollen, auf eine oder mehrere Referenzsequenzen,
- Assemblieren der alignierten Reads, wodurch ein Contig erzeugt wird,
- Vergleichen der einen oder mehreren Referenzsequenzen und des Contigs, wodurch Informationen bezüglich der Position von Übereinstimmungsfehlern und des Typs von Übereinstimmungsfehlern zwischen der einen oder mehreren Referenzsequenzen und dem Contig erhalten werden,

• Entropie-Codieren der Informationen bezüglich der Position von Übereinstimmungsfehlern und des Typs von Übereinstimmungsfehlern, und

• wobei die Informationen bezüglich der Position von Übereinstimmungsfehlern und des Typs von Übereinstimmungsfehlern unter Verwendung von jeweils einem ersten Deskriptor (203) und einem zweiten Deskriptor (204) angegeben werden, und der erste Deskriptor und der zweite Deskriptor in derselben Zugriffseinheit eingekapselt sind, um so an der Decodierungsvorrichtung die selektive Rekonstruktion der einen oder der mehreren für das Alignment verwendeten Referenzsequenzen zu ermöglichen.

**7.** Vorrichtung nach Anspruch 6, ferner umfassend Mittel zum Ausführen des Codierungsverfahrens nach einem der Ansprüche 2 bis 5.

**8.** Computerimplementiertes Verfahren zum Decodieren codierter Genomsequenzdaten, umfassend die folgenden Schritte:

Parsen der codierten Eingabedatei, um Zugriffseinheiten von genomischen Daten zu erhalten,

Entropie-Decodieren von Informationen bezüglich der Position und des Typs von Übereinstimmungsfehlern in einem Contig, und

Modifizieren des Contigs durch Einsetzen der Informationen bezüglich der Positionen und Typen von Übereinstimmungsfehlern, wodurch eine oder mehrere Referenzsequenzen erhalten werden, die für das Alignment vor der Komprimierung verwendet wurden, wobei das Modifizieren des Contigs durch Einsetzen der Informationen bezüglich der Positionen und Typen von Übereinstimmungsfehlern, wodurch die eine oder die mehreren Referenzsequenzen erhalten werden, ferner das Entropie-Decodieren eines ersten Deskriptors (203) und eines zweiten Deskriptors (204) umfasst, und ferner das Entkapseln des ersten Deskriptors und des zweiten Deskriptors aus derselben Zugriffseinheit umfasst, um so die selektive Rekonstruktion der einen oder der mehreren Referenzsequenzen zu erhalten.

**9.** Verfahren nach Anspruch 8, ferner umfassend das Decodieren der Länge des Contigs aus einem Syntax-Header, der in der Eingabedatei enthalten ist.

**10.** Verfahren nach Anspruch 8, ferner umfassend eine reverse Binarisierung des ersten Deskriptors, wobei der erste Deskriptor unter Verwendung einer geteilten, einheitsweise abgeschnittenen unären Binarisierung binarisiert wird, wobei die geteilte, einheitsweise abgeschnittene unäre Binarisierung eine Verkettung von wiederholten abgeschnittenen unären Binarisierungen ist, bei welcher jede abgeschnittene unäre Binarisierung auf Abschnitte des zu binarisierenden Werts angewandt wird, die N Bits lang sind, wobei N ein vorgewählter Parameter ist, vorzugsweise ferner umfassend eine reverse Binarisierung des zweiten Deskriptors, woobei der zweite Deskriptor unter Verwendung einer abgeschnittenen unären Binarisierung binarisiert wird, wobei auf den Wert des zweiten Deskriptors eine Null folgt, und wenn der Wert gleich dem höchsten möglichen zu binarisierenden Wert ist, das nachgestellte 0-Bit verworfen wird.

**11.** Verfahren nach Anspruch 8, wobei die Eingabedatei keine Informationen enthält, welche die Verwendung einer spezifischen Referenzsequenz signalisieren.

**12.** Vorrichtung zum Decodieren von Genomsequenzdaten, umfassend Mittel zum:

Parsen der codierten Eingabedatei, um Zugriffseinheiten von genomischen Daten zu erhalten,

Entropie-Decodieren von Informationen bezüglich der Position und des Typs von Übereinstimmungsfehlern in einem Contig, und

Modifizieren des Contigs durch Einsetzen der Informationen bezüglich der Positionen und Typen von Übereinstimmungsfehlern, wodurch eine oder mehrere Referenzsequenzen erhalten werden, die für das Alignment vor der Komprimierung verwendet wurden, wobei das Modifizieren des Contigs durch Einsetzen der Informationen bezüglich der Positionen und Typen von Übereinstimmungsfehlern, wodurch die eine oder die mehreren Referenzsequenzen erhalten werden, ferner das Entropie-Decodieren eines ersten Deskriptors (203) und eines zweiten Deskriptors (204) umfasst, und ferner das Entkapseln des ersten Deskriptors und des zweiten Deskriptors aus derselben Zugriffseinheit umfasst, um so die selektive Rekonstruktion der einen oder der mehreren Referenzsequenzen zu erhalten.

**13.** Vorrichtung nach Anspruch 12, ferner umfassend Mittel zum Ausführen des Decodierungsverfahrens nach einem der Ansprüche 9 bis 11.

**14.** Computerlesbares Medium, das Anweisungen speichert, die, wenn sie von einem oder mehreren Prozessoren ausgeführt werden, den einen oder die mehreren Prozessoren veranlassen, das Verfahren nach einem der Ansprüche 1 bis 5 durchzuführen.

**15.** Computerlesbares Medium, das Anweisungen speichert, die, wenn sie von einem oder mehreren Prozessoren ausgeführt werden, den einen oder die mehreren Prozessoren veranlassen, das Verfahren nach einem der Ansprüche 8 bis 11 durchzuführen.

**Revendications**

**1.** Procédé mis en oeuvre par ordinateur pour encoder des données de séquences génomiques alignées comprenant des lectures de séquences de nucléotides, ledit procédé comprenant les étapes :

- d'alignement desdites lectures à une ou plusieurs séquences de référence créant ainsi des lectures alignées,
- de mise en correspondance, sur lesdites une ou plusieurs séquences de référence, desdites lectures alignées devant être codées,
- d'assemblage desdites lectures alignées créant ainsi un contig,
- de comparaison desdites une ou plusieurs séquences de référence et dudit contig obtenant ainsi des informations concernant la position de mésappariements et le type de mésappariements entre les une ou plusieurs séquences de référence et le contig,
- de codage par entropie desdites informations concernant la position de mésappariements et le type des mésappariements, et
- dans lequel lesdites informations concernant la position de mésappariements et le type de mésappariements sont indiquées en utilisant respectivement un premier descripteur (203) et un deuxième descripteur (204), et ledit premier descripteur et le deuxième descripteur sont encapsulés dans une même unité d'accès de manière à permettre, sur le dispositif de décodage, la reconstruction sélective des une ou plusieurs séquences de référence utilisées pour l'alignement.

**2.** Procédé selon la revendication 1, dans lequel l'assemblage desdites lectures alignées comprend l'étape de sélection, pour chaque position des une ou plusieurs séquences de référence, du nucléotide, qui est présent avec la fréquence la plus élevée dans les lectures alignées sur cette position.

**3.** Procédé selon la revendication 1 ou 2, dans lequel la longueur dudit contig est définie en tant que paramètre d'entrée dans l'encodeur ou adapté de manière dynamique par l'encodeur, de manière préférée ledit premier descripteur est binarisé en utilisant une binarisation SUTU [Split Unit-wise Truncated Unary binarization], dans lequel ledit SUTU est une concaténation de binarisations unaires tronquées répétées, où chaque binarisation unaire tronquée est appliquée à des parties de la valeur devant être binarisée, qui présentent une longueur de N bits, dans lequel N est un paramètre présélectionné, dans lequel ladite longueur dudit contig est contenue dans un en-tête syntaxique.

**4.** Procédé selon la revendication 3, dans lequel la longueur dudit contig est contenue dans un en-tête syntaxique et ledit premier descripteur est binarisé en utilisant une binarisation SUTU, dans lequel SUTU est une concaténation de binarisations unaires tronquées répétées, où chaque binarisation unaire tronquée est appliquée à des parties de valeur devant être binarisée qui présentent une longueur de N bits, dans lequel N est un paramètre présélectionné, et dans lequel ledit deuxième descripteur est binarisé en utilisant une binarisation unaire tronquée, dans lequel la valeur dudit deuxième descripteur est suivie d'un zéro et, si ladite valeur est égale à la valeur la plus grande possible devant être binarisée, le bit 0 de suivi est éliminé.

**5.** Procédé selon la revendication 3, dans lequel ledit procédé n'encode pas des informations signalant l'utilisation d'une séquence de référence spécifique.

**6.** Appareil d'encodage de données de séquences génomiques, lesdites données de séquences génomiques comprenant des lectures de séquences de nucléotides, ledit appareil comprenant des moyens pour :

- aligner lesdites lectures à une ou plusieurs séquences de référence créant ainsi des lectures alignées,
- mettre en correspondance, sur lesdites une ou plusieurs séquences de référence lesdites lectures alignées devant être codées,
- assembler lesdites lectures alignées créant ainsi un contig,

- comparer lesdites une ou plusieurs séquences de référence et ledit contig en obtenant ainsi des informations concernant la position de mésappariements et le type de mésappariements entre les une ou plusieurs séquences de référence et le contig,

- coder par entropie lesdites informations concernant la position du mésappariement et le type de mésappariements, et

- dans lequel lesdites informations concernant la position de mésappariements et le type de mésappariement sont indiquées en utilisant respectivement un premier descripteur (203) et un deuxième descripteur (204), et ledit premier descripteur et le deuxième descripteur sont encapsulés dans la même unité d'accès de manière à permettre, sur le dispositif de décodage, la reconstruction sélective des une ou plusieurs séquences de référence utilisées pour l'alignement.

7. Appareil selon la revendication 6, comprenant en outre des moyens pour exécuter le procédé d'encodage selon l'une quelconque des revendications 2 à 5.

8. Procédé mis en oeuvre par ordinateur pour décoder des données de séquences génomiques encodées, comprenant les étapes :

d'analyse syntaxique du fichier d'entrée encodé de manière à obtenir des unités d'accès de données génomiques,

de décodage par entropie d'informations concernant la position d'un mésappariement et le type de mésappariement dans un contig, et

de modification du contig en employant lesdites informations concernant des positions et des types de mésappariements en obtenant ainsi une ou plusieurs séquences de référence utilisées pour l'alignement avant la compression, dans lequel la modification du contig par l'emploi desdites informations concernant des positions et types de mésappariements en obtenant ainsi lesdites une ou plusieurs séquences de référence comprend en outre le décodage par entropie d'un premier descripteur (203) et d'un deuxième descripteur (204), et comprenant en outre la décapsulation de la même unité d'accès dudit premier descripteur et du deuxième descripteur de manière à obtenir la reconstruction sélective desdites une ou plusieurs séquences de référence.

9. Procédé selon la revendication 8, comprenant en outre le décodage de la longueur dudit contig d'un en-tête syntaxique contenu dans le fichier d'entrée.

10. Procédé selon la revendication 8, comprenant en outre une binarisation inverse dudit premier descripteur, dans lequel ledit premier descripteur est binarisé en utilisant une binarisation SUTU, dans lequel SUTU est une concaténation de binarisations unaires tronquées répétées, où chaque binarisation unaire tronquée est appliquée à des parties de la valeur à binariser qui présentent une longueur de N bits, dans lequel N est un paramètre présélectionné, de manière préférée comprenant en outre une binarisation inverse dudit deuxième descripteur, dans lequel ledit deuxième descripteur est binarisé en utilisant une binarisation unaire tronquée, dans lequel la valeur dudit deuxième descripteur est suivie d'un zéro et, si ladite valeur est égale à la valeur la plus grande possible devant être binarisée, le bit 0 de suivi est éliminé.

11. Procédé selon la revendication 8, dans lequel ledit fichier d'entrée ne contient pas des informations signalant l'utilisation d'une séquence de référence spécifique.

12. Appareil pour le décodage de données de séquences génomiques encodées, comprenant des moyens pour :

l'analyse syntaxique du fichier d'entrée encodé de manière à obtenir des unités d'accès de données génomiques,

de décodage par entropie des informations concernant la position d'un mésappariement et le type de mésappariement dans un contig, et

de modification du contig en employant lesdites informations concernant des positions et des types de mésappariements en obtenant ainsi une ou plusieurs séquences de référence utilisées pour l'alignement avant la compression, dans lequel la modification du contig par l'emploi desdites informations concernant des positions et des types de mésappariements en obtenant ainsi lesdites une ou plusieurs séquences de référence comprend en outre le décodage par entropie d'un premier descripteur (203) et d'un deuxième descripteur (204), et comprenant en outre la décapsulation de la même unité d'accès dudit premier descripteur et du deuxième descripteur de manière à obtenir la reconstruction sélective desdites une ou plusieurs séquences de référence.

13. Appareil selon la revendication 12, comprenant en outre des moyens pour exécuter le procédé de décodage selon l'une quelconque des revendications 9 à 11.

14. Support lisible par ordinateur stockant des instructions, qui, lorsqu'elles sont exécutées par un ou plusieurs processeurs, amènent lesdits un ou plusieurs processeurs, à réaliser le procédé selon l'une quelconque des revendications 1 - 5.

15. Support lisible par ordinateur stockant des instructions, qui, lorsqu'elles sont exécutées par un ou plusieurs processeurs, amènent lesdits un ou plusieurs processeurs à réaliser le procédé selon l'une quelconque des revendications 8-11.

Figure 1.

Figure 2.

**Figure 3.**

EP 3 583 249 B1

**Figure 4.**

**Figure 5.**

$\Delta i$ = i[th] genomic interval on the reference sequence
AU$i$ = i[th] Access Unit encapsulating compressed descriptors
representing sequences mapped in interval $\Delta i$

**Figure 6.**

Pos data blocks Rcomp data blocks Pair data blocks Rlen data blocks

| Header | Pos1 | ... | PosN | Rc1 | ... | RcN | Pair1 | ... | PairN | Rlen1 | ... | RlenN | Flags |
|--------|------|-----|------|-----|-----|-----|-------|-----|-------|-------|-----|-------|-------|

Pos block    Rcomp block    Pair block    Rlen block

791    792    793    794    795    796    790

Figure 7.

Coordinate system on a
«Reference Sequence»

Figure 8.

# Half mapped

Unknown region (gap)

Reference                    Reference

Assembled contig

Previously unmapped pair

**Figure 9.**

EP 3 583 249 B1

| 5 | 7 | 12 | 13 | 15 |
|---|---|----|----|----|
| C | T | T | C | A |

← positions in local assembly are coded using genomic descriptor **rftp**.

← different nucleotides coded using genomic descriptor **rftt**

| A | C | G | T | N |
|---|---|---|---|---|
| 0 | 1 | 2 | 3 | 4 |

1000

1001

1004

transformation

1002

1003

| 5 | 2 | 5 | 1 | 2 |
|---|---|---|---|---|
| 1 | 3 | 3 | 1 | 0 |

1005

1006

binarization

| 11110 | 110 | 11110 | 10 | 110 |
|-------|-----|-------|----|-----|
| 111 | 0 | 0 | 0 | 10 |

entropy coding

1008

1007

1009

**Figure 10.**

Genomic sequences
generated by sequencing
devices

Reference
genome

| T | A | G | T | A | C | A | G | T | C | G | C | G | T | A | C | G | A | G | G | A | C | T | C | A | C |

Genomic sequences
mapped on the reference
genome

no sequences
map in this
region

| T | A | C | C | G | T | C | G |

no sequences
map in this
region

| C | G | G | A | C | A | C | A |

no sequences
map in this
region

| $AU_n$ | $AU_{n+1}$ | $AU_{n+1}$ | $AU_{n+1}$ | $AU_{n+1}$ |

these Access Units will contain a compressed
representation of those regions of the reference
genome where no sequences are mapped

**Figure 11.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VOGES, J. ; MUNDERLOH, M. ; OSTERMANN, J.** Predictive Coding of Aligned Next-Generation Sequencing Data. *2016 Data Compression Conference (DCC)*
- **BENOIT, G. et al.** Reference-free compression of high throughput sequencing data with a probabilistic de Bruijn graph. *BMC Bioinformatics,* 2015, vol. 16, 288
- **MIHAI POP et al.** Comparative genome assembly. *Briefings in bioinformatics,* 01 September 2004, 237-248
- **K. SCHNEEBERGER et al.** Reference-guided assembly of four diverse Arabidopsis thaliana genomes. *PROCEEDINGS OF THE NATIONAL ACADEMY OF SCIENCES,* 21 June 2011, vol. 108, 10249-10254
- *CRAM format specification (version 3.0),* 08 September 2016
- Comparison of high-throughput sequencing data compression tools. **NUMANAGIC, I. et al.** Nature Methods. Nature Publishing Group, 2016, vol. 13, 1005-1008